# EUROPEAN PATENT SPECIFICATION

(11) **EP 1 429 804 B1**
(45) Date of publication and mention of the grant of the patent: **19.05.2010**
(21) Application number: 02762584.7
(22) Date of filing: 27.09.2002
(51) Int. Cl.: A61K 38/17, A61K 39/395

(54) **INHIBITORS OF COSTIMULATION BY OX40 AND THEIR USE AGAINST VIRUS INDUCED IMMUNOPATHOLOGY**
INHIBITOREN DER KOSTIMULATION VIA OX40 UND DEREN VERWENDUNG GEGEN EINE VON VIRUSEN INDUZIERTEN IMMUNPATHOLOGIE
INHIBITEURS DE LA COSTIMULATION PAR OX40 ET LEUR UTILISATION CONTRE DES IMMUNOPATHOLOGIES INDUCÉES PAR DES VIRUSES

(30) Priority: 27.09.2001 GB 0123276
(43) Date of publication of application: 23.06.2004
(73) Proprietor: IMPERIAL COLLEGE INNOVATIONS LIMITED, London SW7 2AZ (GB)
(72) Inventor: HUSSELL, Tracey, Imperial College Innovations Ltd., London SW7 2AZ (GB); HUMPHREYS, Ian, Imperial College Innovations Ltd., London SW7 2AZ (GB)
(74) Representative: Lee, Nicholas John
(86) International application number: PCT/GB2002/004386
(87) International publication number: WO 2003/026693

(56) References cited:
- WO-A-95/12673
- Y. TAKAHASHI ET AL.: "OX40 stimulation by gp34/OX40 ligand enhances productive human immunodeficiency virus type 1 infection." JOURNAL OF VIROLOGY, vol. 75, no. 15, August 2001 (2001-08), pages 6748-6757, XP002254083 Baltimore, MD, USA
- H. AKIBA ET AL.: "Critical contribution of OX40 ligand to T helper cell type 2 differentiation in experimental leishmaniasis." THE JOURNAL OF EXPERIMENTAL MEDICINE, vol. 191, no. 2, 17 January 2000 (2000-01-17), pages 375-380, XP002254084 New York, NY, USA
- L. HIGGINS ET AL.: "Regulation of T cell activation in vitro and in vivo by targeting the OX40-OX40 ligand interaction: Amelioration of ongoing inflammatory bowel disease with an OX40-IgG fusion protein, but not with an OX40 ligand-IgG fusion protein." THE JOURNAL OF IMMUNOLOGY, vol. 162, no. 1, 1 January 1999 (1999-01-01), pages 486-493, XP002254085 Baltimore, MD, USA cited in the application

## Description

This invention relates to uses of agents, such as biological products which can interact with immune system components, to treat or ameliorate diseases; to materials and compositions useful for such purposes; and to their preparation and the preparation of components thereof. Among these uses are uses as adjuvants for vaccines directed against viruses.

OX40 (also known as ACT-4, CD134 and Tumour Necrosis Factor Receptor Superfamily member 5) is a cell surface receptor that can occur on the surface of activated CD4⁺ T-cells. WO95/12673 describes human OX40, and discloses an isolated form of OX-40, as well as fragments of OX40 and biologically-active derivatives of OX40, e.g. fusion proteins such as a fusion protein comprising an OX40 extracellular domain sequence fused with the sequence of an IgG heavy-chain constant-region domain. It also discloses methods of using binding agents related to OX40, e.g. antibodies that bind to OX40, to alter immune responses.

It has been proposed to abrogate harmful cell-mediated immunopathological responses by using OX40:Ig fusion protein (or anti-OX40 ligand antibodies) to block the interaction between the T cell and antigen-presenting cell (APC). Using this approach, several authors have reported improving colitis in SCID mice, inflammatory bowel disease, allergic encephalomyelitis, acute graft versus host disease and collagen-induced arthritis.

Anti-inflammatory activity was reported in particular by Higgins et al (1999) J Immunol 162:486-93. Further information about OX40 and related materials is contained in this paper and in published literature cited therein.

OX40 stimulation by the OX40 ligand has been shown to enhance HIV-1 replication in CD4+ T cells which had been induced to over-express OX40, *in vitro* (Takahashi et al (2001) Journal of Virology 75:6748-6757). The OX40:OX40 ligand interaction has also been shown to play a role in the development of Th2 cells during *Leishmania major* infection (Akiba et al (2000) The Journal of Experimental Medicine 191:375-380.).

It is one of the aims of the present invention to provide new treatments for inflammatory disease conditions. Further aims and aspects of the invention are indicated by the description given hereinbelow.

In one of its aspects, the invention provides a composition comprising one or more OX40 polypeptides or biologically active fragments thereof, or antibodies which bind to OX40, that can inhibit immune system costimulation by OX40, for use in treating, ameliorating or preventing virus induced immunopathology.

Also disclosed herein are methods for the treatment, amelioration or prevention of diseases associated with virus, bacteria or other infective agent, comprising administering to a subject one or more compounds that can inhibit immune system costimulation by OX40.

Viral infections suitable for treatment by the medicaments of the present invention include infection with influenza or RSV or related viruses (e.g. negative-stranded viruses with segmented genomes: for which see e.g. WO 91/03552). In particular embodiments, the invention is useful in relation to prevention or reduction of lung inflammation caused by influenza virus and/or by respiratory syncytial virus (RSV). The use of the composition of the present invention for preventing or reducing lung inflammation caused by fungal infection by e.g. *C neoformans* is also disclosed herein.

Illnesses induced by infection of the lung by RSV and influenza virus are considered to be caused by the recruitment of an excessive inflammatory infiltrate. Reduction of this infiltrate by, for example, TNF depletion can eliminate weight loss, cachexia and immobility in infected animals. During infection, both CD4⁺ and CD8⁺ cells are believed to be recruited, which secrete large amounts of IFN-gamma.

According to one aspect of the invention, it has been found that inhibition of OX40 costimulation, e.g. by a fusion protein based on OX40 and an immunoglobulin, preferably IgG, can prevent or ameliorate virus-induced immunopathology, e.g. pathology associated with infection by influenza or RSV virus or related viruses, e.g. in the lung. The fusion protein may comprise the extracellular domain of human OX40 linked via its C-terminal to the N-terminal of the constant domain of a human immunoglobulin (preferably the heavy chain thereof, and more preferably the hinge, CH2 and CH3 regions thereof). The human immunoglobulin may be IgGγ. Accordingly, substances that are able to inhibit OX40 costimulation can prevent or ameliorate such pathology, and can be useful in pharmaceutical preparations for the treatment or management of diseases in which such pathology is a feature. Additionally, it has been found that such a fusion protein can ameliorate lung infection by *C. neoformans.*

The invention also provides a product containing the composition of the invention and an anti-inflammatory drug as a combined preparation for simultaneous, sequential or separate use in treating, ameliorating or preventing virus induced immunopathology.

Also disclosed herein is a composition comprising a vaccine and one or more compounds that can inhibit immune system costimulation by OX40. The vaccine may be an anti-viral or antibacterial vaccine.

OX40:IgG fusion proteins and biologics with similar binding activity are also provided as vaccine adjuvants for vaccines against viruses and bacteria. The adjuvants can be given either at the same time as or after the vaccines themselves. Vaccines against viruses, such as against influenza or RSV or related viruses, with which the adjuvants can be used in combination or association, include conventional vaccines directed against such viruses. Generally the range of vaccines with which the adjuvants can be associated or combined includes killed vaccines, subunit vaccines and genetically disabled vaccines as for example those disclosed in relation to influenza viruses and related viruses in WO 91/03552. Thus, a product containing an anti-viral vaccine and one or more compounds that can inhibit immune system costimulation by OX40 as a combined preparation for simultaneous, sequential or separate use in vaccination against said virus is also disclosed herein.

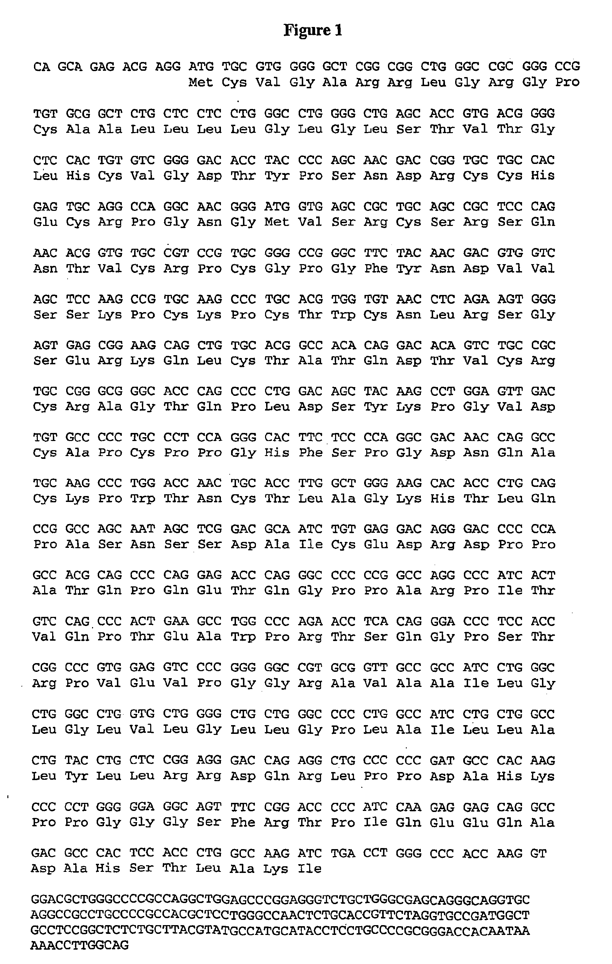

Compounds that can inhibit immune system costimulation by OX40 include proteins related to OX40 that have this ability. Examples of such proteins are described in WO 95/12673. Thus, the compound may be human OX40, the amino acid and nucleic acid sequences of which is shown in Figure 1, as well as other proteins that represent allelic, nonallelic, and higher cognate variants of OX40, and natural or induced mutants of any of these. Usually, OX40 polypeptides will also show substantial sequence identity with the sequence of Figure 1. Typically, an OX40 polypeptide will contain at least 4 and more commonly 5, 6, 7, 10 or 20, 50 or more contiguous amino acids from the sequence of Figure 1. It is well known in the art that functional domains, such as binding domains or epitopes can be formed from as few as four amino acids residues.

The following terms are used to describe the sequence relationships between two or more polynucleotides: "reference sequence", "comparison window", "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA or gene sequence given in a sequence listing, such as a polynucleotide sequence shown in Figure 1, or may comprise a complete cDNA or gene sequence. Generally, a reference sequence is at least 20 nucleotides in length, frequently at least 25 nucleotides in length, and often at least 50 nucleotides in length. Since two polynucleotides may each (1) comprise a sequence (i.e., a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window", as used herein, refers to a conceptual segment of at least 20 contiguous nucleotide positions wherein a polynucleotide sequence may be compared to a reference sequence of at least 20 contiguous nucleotides and wherein the portion of the polynucleotide sequence in the comparison window may comprise additions or deletions (i.e., gaps) of 20 % or less as compared to the reference sequence (which does not comprise additions or deletions) for optimal alignment of the two sequences. Optimal alignment of sequences for aligning a comparison window may be conducted by the local homology algorithm of Smith & Waterman, Appl. Math. 2:482 (1981), by the homology alignment algorithm of Needleman & Wunsch, J. Mol. Biol. 48:443 (1970), by the search for similarity method of Pearson & Lipman, Proc. Natl. Acad. Sci. (USA) 85:2444 (1988), by computerized implementations of these algorithms (FASTDB (Intelligenetics), BLAST (National Center for Biomedical Information) or GAP, BESTFIT, FASTA, and TFASTA (Wisconsin Genetics Software Package Release 7.0, Genetics Computer Group, 575 Science Dr., Madison, Wis.)), or by inspection, and the best alignment (i.e., resulting in the highest percentage of sequence similarity over the comparison window) generated by the various methods is selected. The term "sequence identity" means that two polynucleotide sequences are identical (i.e., on a nucleotide-by-nucleotide basis) over the window of comparison. The term "percentage of sequence identity" is calculated by comparing two optimally aligned sequences over the window of comparison, determining the number of positions at which the identical nucleic acid base (e.g., A, T, C, G, U, or I) occurs in both sequences to yield the number of matched positions, dividing the number of matched positions by the total number of positions in the window of comparison (i.e., the window size), and multiplying the result by 100 to yield the percentage of sequence identity. The terms "substantial identity" as used herein denotes a characteristic of a polynucleotide sequence, wherein the polynucleotide comprises a sequence that has at least 70, 80 or 85 % sequence identity, preferably at least 90 to 95 % sequence identity, more usually at least 99 % sequence identity as compared to a reference sequence over a comparison window of at least 20 nucleotide positions, frequently over a window of at least 25-50 nucleotides, wherein the percentage of sequence identity is calculated by comparing the reference sequence to the polynucleotide sequence which may include deletions or additions which total 20 percent or less of the reference sequence over the window of comparison.

As applied to polypeptides, the term "substantial identity" means that two peptide sequences, when optimally aligned, such as by the programs BLAZE (Intelligenetics) GAP or BESTFIT using default gap weights, share at least 70 % or 80 % sequence identity, preferably at least 90 % sequence identity, more preferably at least 95 % sequence identity or more (e.g., 99 % sequence identity). Preferably, residue positions which are not identical differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine.

The term "higher cognate variants" as used herein refers to a gene sequence that is evolutionarily and functionally related between humans and higher mammalian species, such as primates, porcines and bovines. The term does not include gene sequences from rodents, such as rats.

OX40 polypeptides will typically exhibit substantial amino acid sequence identity with the amino acid sequence of Figure 1, and be encoded by nucleotide sequences that exhibit substantial sequence identity with the nucleotide sequence of Figure 1. Such nucleotides will also typically hybridise to the sequence of Figure 1 under stringent conditions. Stringent conditions are sequence dependent and will be different in different circumstances. Generally, stringent conditions are selected to be about 5°C lower than the thermal melting point (Tm) for the specific sequence at a defined ionic strength and pH. The Tm is the temperature (under defined ionic strength and pH at which 50% of the target sequence hybridises to a perfectly matched probe. Typically, stringent conditions will be those in which the salt concentration is at least about 0.02 molar at pH 7 and the temperature is at least about 60°C. As other factors may significantly affect the stringency of hybridisation, including, among others, base composition and size of the complementary strands, the presence of organic solvents and the extent of base mismatching, the combination of parameters is more important than the absolute measure of any one.

Usually, OX40 polypeptides will share at least one antigenic determinant in common with a protein having the sequence of Figure 1. The existence of a common antigenic determinant is evidenced by cross-reactivity of the variant protein with any antibody prepared against a protein having the sequence of Figure 1. Cross-reactivity is often tested using polyclonal sera against a protein having the sequence of Figure 1, but can also be tested using one or more monoclonal antibodies against a protein having the sequence of Figure 1, such as the antibody designated L106 which is produced by the hybridoma line HBL106, deposited as ATCC HB 11483, (at the American Type Culture Collection, 12301 Parklawn Drive, Rockville, Md. 20852, on 3 Nov. 1993).

Polypeptides useful in the present invention may contain modified polypeptide backbones. Modifications include chemical derivatisations of polypeptides, such as acetylations, carboxylations and the like. They also include glycosylation modifications (N- and O-linked) and processing variants of a typical polypeptide.

The amino acid sequence shown in Figure 1 contains a 22 or 24 amino acid putative N-terminal signal sequence. It also contains a single additional hydrophobic stretch of 27 amino acids spanning residues 213-240. The hydrophobic stretch probably corresponds to a transmembrane domain. The 189 or 191 amino acids (depending on the exact location of the signal cleavage site) of the Figure 1 sequence amino-proximal to the transmembrane segment are designated the extracellular domain, while the 37 amino acids carboxy-proximal to the transmembrane segment are designated the intracellular domain. From the amino-terminus, the extracellular domain has an amino-terminal hydrophobic putative signal sequence, and three intrachain loops formed by disulphide bonding between paired cysteine residues.

Although not all of the domains discussed above are necessarily present in proteins useful in the invention, an extracellular domain is expected to be present in most. Indeed, in some proteins, only an extracellular domain is present, and the natural state of such proteins is not as cell-surface bound proteins, but as soluble proteins, for example, dispersed in an extracellular body fluid.

Besides substantially full-length polypeptides, biologically-active fragments of the polypeptides are useful in the present invention. Significant biological activities include antagonism of the binding of an OX40 polypeptide to its ligand. A segment of an OX40 protein or a domain thereof will ordinarily comprise at least about 5, 7, 9, 11, 13, 16, 20, 40, or 100 contiguous amino acids. Some fragments will contain only extracellular domains, such as one or more disulphide-bonded loops. Such fragments will often retain the binding specificity of an intact OX40 polypeptide, but will be soluble rather than membrane bound.

Fragments or analogues comprising substantially one or more functional domain (e.g., an extracellular domain) of OX40 and related peptides can be fused to heterologous polypeptide sequences, such that the resultant fusion protein exhibits the functional property(ies) conferred by the OX40 receptor fragment and/or the fusion partner.

Recombinant globulins (Rg) formed by fusion of OX40 fragments and immunoglobulin components often have most or all of the physiological properties associated with the constant region of the particular immunoglobulin class used. For example, the recombinant globulins may be capable of fixing complement, mediating antibody dependent cell toxicity, stimulating B cells, or traversing blood vessel walls and entering the interstitial space. The recombinant globulins are usually formed by fusing the C-terminus of an OX40 extracellular domain to the N-terminus of the constant region domain of a heavy chain immunoglobulins, thereby simulating the conformation of an authentic immunoglobulin chain. The immunoglobulin chain is preferably of human origin. Recombinant globulins are usually soluble and may have a prolonged serum half-life, the capacity to lyse target cells for which OX40 receptor has affinity, by effector functions, and the capacity to bind molecules such as protein A and G, which can be used to immobilise the recombinant globulin in binding analyses.

Also suitable for use in the present invention are antibodies which bind to OX40. Such antibodies can be produced by a variety of means. The production of non-human monoclonal antibodies, e.g., murine, rat and so forth, is well known. Several techniques for generation of human monoclonal antibodies have also been described, see, e.g., U.S. Pat. No. 5,001,065. One technique that has successfully been used to generate human monoclonal antibodies against a variety of antigens is the trioma methodology of Ostberg et al. (1983), Hybridoma 2:361-367, U.S. Pat. No. 4,634,664, and U.S. Pat. No. 4,634,666. The antibody-producing cell lines obtained by this method are called triomas, because they are descended from three cells: two human and one mouse. Triomas have been found to produce antibody more stably than ordinary hybridomas made from human cells. An alternative approach is the generation of humanised immunoglobulins by linking the CDR regions of non-human antibodies to human constant regions by recombinant DNA techniques (Queen et al., Proc. Natl. Acad. Sci. USA 86:10029-10033 (1989) and WO 90/07861). The humanised immunoglobulins have variable region framework residues substantially from a human immunoglobulin (termed an acceptor immunoglobulin) and complementarity determining regions substantially from a mouse immunoglobulin, e.g., the L106 antibody (referred to as the donor immunoglobulin). The constant region(s), if present, are also substantially from a human immunoglobulin. The human variable domains are usually chosen from human antibodies whose framework sequences exhibit a high degree of sequence identity with the murine variable region domains from which the CDRs were derived. The heavy and light chain variable region framework residues can be derived from the same or different human antibody sequences. The human antibody sequences can be the sequences of naturally occurring human antibodies or can be consensus sequences of several human antibodies (WO 92/22653). Certain amino acids from the human variable region framework residues are selected for substitution based on their possible influence on CDR conformation and/or binding to antigen. Investigation of such possible influences is by modelling, examination of the characteristics of the amino acids at particular locations, or empirical observation of the effects of substitution or mutagenesis of particular amino acids.

A further approach for isolating DNA sequences which encode a human monoclonal antibody or a binding fragment thereof is by screening a DNA library from human B cells according to the general protocol outlined by Huse et al., Science 246:1275-1281 (1989) and then cloning and amplifying the sequences which encode the antibody (or binding fragment) of the desired specificity. The protocol described by Huse is rendered more efficient in combination with phage display technology. See, e.g., WO 91/17271 and WO 92/01047. Phage display technology can also be used to mutagenise CDR regions of antibodies previously shown to have affinity for ACT-4 receptors or their ligands. Antibodies having improved binding affinity are selected.

Anti-OX40 antibodies that specifically bind to the same epitope as the L106 antibody are usually identified by a competitive binding assay. The assay has three components, an OX40 polypeptide, L106 antibody, which is usually labelled, and the antibody under test. The test antibody binds to the same epitope as the L106 antibody if it reduces the amount of L106 antibody that specifically binds to OX40. Antibodies binding to the same epitope as L106 may exhibit a substantially, but not completely, identical amino acid sequence to the L106 antibody, or may have an unrelated primary structure to the L106 antibody.

Anti-OX40 antibodies having a different binding specificity than L106 (i.e., which bind to a different epitope) are identified by a complementary approach. Test antibodies are screened for failure to compete with the L106 antibody for binding to an OX40 polypeptide. The extent of screening can be reduced by generating antibodies with a protocol in which a fragment lacking a specific epitope bound by L106 is used as an immunogen.

Fragments of such antibodies may be used. Antibody fragments include separate heavy chains, light chains Fab, Fab' F(ab')², Fabc, and Fv. Fragments are produced by recombinant DNA techniques, or by enzymic or chemical separation of intact immunoglobulins.

Other agents suitable for use in the invention may be found by screening for OX40 antagonists. Compounds that block OX40-induced DNA synthesis or protein phosphorylation are antagonists. Antagonistic activity can also be determined from other functional or physical endpoints of leukocyte activation, or from clinically desirable or undesirable outcomes, such as cytolytic activity, or extravasation of leukocytes into tissues from blood vessels.

The ability of agents to antagonise T-cell proliferation *in vitro* can be correlated with the ability to affect the immune response *in vivo.* In vivo activity is typically assayed using suitable animal models such as mice or rats.

The form of medicament of the invention depends on the intended mode of administration. The compositions may also include, depending on the formulation desired, pharmaceutically-acceptable, non-toxic carriers or diluents, which are defined as vehicles commonly used to formulate pharmaceutical compositions for animal or human administration. The diluent is selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like.

Amounts effective for use in the present invention will depend upon the severity of the condition, the general state of the patient, and the route of administration, and combination with other immunosuppressive drugs, if any, but generally range from about 10 ng to about 1 g of active agent per dose, with single dosage units of from 10 mg to 100 mg per kg patient being commonly used. Pharmaceutical compositions can be administered systemically by intravenous infusion, or locally by injection.

In prophylactic applications, pharmaceutical compositions are administered to a patients at risk of, but not already suffering an undesired immune reaction. The amount of antibody to be administered is a "prophylactically effective dose," the precise amounts of which will depend upon the patient's state of health and general level of immunity, but generally range from 10 ng to 1 g per dose, especially 10 mg to 100 mg per patient.

The therapeutic agents of the present invention can also be combined with traditional therapeutics, and can be used to lower the dose of such agents to levels below those associated with side effects. For example, other immunosuppressive agents such as antibodies to the α3 domain, T cell antigens (e.g., OKT4 and OKT3), antithymocyte globulin, as well as chemotherapeutic agents such as cyclosporine, glucocorticoids, azathioprine, prednisone can be used in the present invention.

A pharmaceutical composition used in the present connection for treatment of an (human or non-human) animal, preferably mammal, for example to reduce lung inflammation associated with infection by RSV or influenza virus or viruses related thereto, is for example a sterile injectable aqueous formulation comprising an OX40:IgG fusion protein wherein the origin of the sequences of the OX40 and the IgG in the fusion protein is from the same (human or non-human) animal species as the species to be treated, in an amount in for example the range of about 10 µg to about 500 µg, e.g. of the order of about 100 µg. Such dosages have produced effects as described for example in the experimental report below, and dosages of these and other alternative binding agents that can be used in accordance with the present invention can be determined by dose optimisation to achieve these or other desired levels of activity.

Preferred features of each aspect of the invention are as for each of the other aspects *mutatis mutandis.* The prior art documents mentioned herein are incorporated to the fullest extent permitted by law.

The invention will be described further in the following non-limiting example. Reference is made to the accompanying drawings in which:
Figure 1 shows the amino acid and nucleic acid sequences of human OX40;
Figure 2 is a graph illustrating the mean % of original weight after infection for influenza-infected mice when treated with OX40:Ig (open symbols) or with PBS (closed symbols);
Figure 3 is a graph illustrating cumulative illness (scored using an established grading system based on degree of cachexia and mobility) after infection for OX40:Ig treated mice (open bars) and control mice (closed bars);
Figure 4 is a graph illustrating total numbers of CD4⁺ and CD8⁺ T cells infiltrated into bronchoalveolar lavage (BAL) during influenza infection for OX40:Ig treated mice (open symbols)and with control animals (closed symbols);
Figure 5 is a graph illustrating the mean % of original weight after infection for RSV-infected mice when treated with OX40:Ig (open symbols) or with PBS (closed symbols);
Figure 6 is a graph illustrating total numbers of lymphocytes CD4⁺ and CD8⁺ T cells infiltrated into bronchoalveolar lavage (BAL) during RSV infection for OX40:Ig treated mice (open symbols)and with control animals (closed symbols);

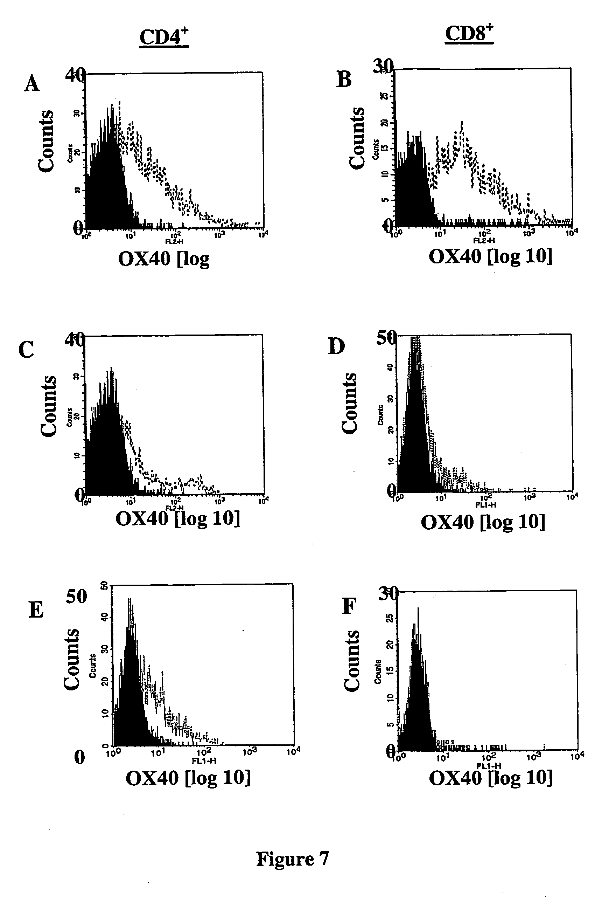

Figure 7: OX40 was up-regulated on CD4⁺ and CD8⁺ T cells during infection. Cells from the lungs of mock (solid histogram) or influenza (a,b), RSV (c,d) or *C. neoformans* (d,e) (dashed line) infected mice were stained with anti-CD4, -CD8 and - OX40 antibodies for 30 minutes on ice and then fixed. Expression was analysed by flow cytometry collecting data on 30,000 lymphocytes. The level of expression of OX40 on cells from mock infected mice was indistinguishable from the isotype matched control stained sample which has been omitted for clarity. These plots are representative of 5 mice per group in two individual experiments;
Figure 8: OX40L:Ig enhanced fungal clearance, reduced pulmonary eosinophils and enhanced CD4⁺ T cells producing IFNγ.
   C57BL/6 mice (n=5) were infected with *C. neoformans* and treated with PBS (■) or OX40L:Ig (□) on days 0, 2, 5 and 7 of infection. 12 days after infection the number of cfu/lung was determined from lung homogenates (a). BAL eosinophils were enumerated in H and E stained cytocentrifuge preparations, each point representing an individual mouse (b). The frequency of IFN-γ (c) and IL-5 (d) producing cells in the lung was assessed by ELISPOT assay (mean and standard deviation of 5 mice per group). IFNγ in CD4⁺ T cells was evaluated by intracellular cytokine staining in Cryptococcus infected control (e) and OX40L:Ig (f) treated mice. These results are representative of two experiments containing 5 mice per group;

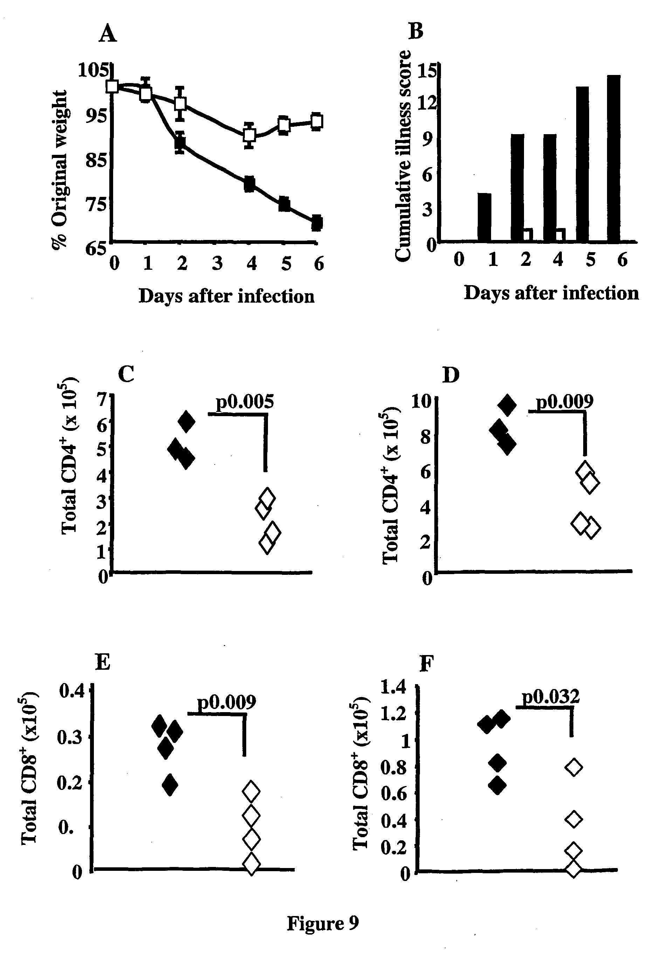

Figure 9: OX40:Ig treatment prevented illness and over exuberant immune responses in RSV and influenza infected BALB/c mice.
   Two groups of 4 mice were infected with influenza virus X31, one group (□) was treated with OX40:Ig on days 0, 2 and 5 of infection. Weight loss was monitored daily and expressed as a percentage of original body weight (a). Illness was scored from 1-5 based on the degree of immobility, cachexia and ruffled fur. The cumulative illness score was calculated by treatment group (b). CD4⁺ (c and d) and CD8⁺ (e and f) T cells in the airways of influenza (c and e) and RSV (d and f) infected mice were determined by multiplying the percent positive cells by flow cytometry with the total viable cells recovered from the BAL;

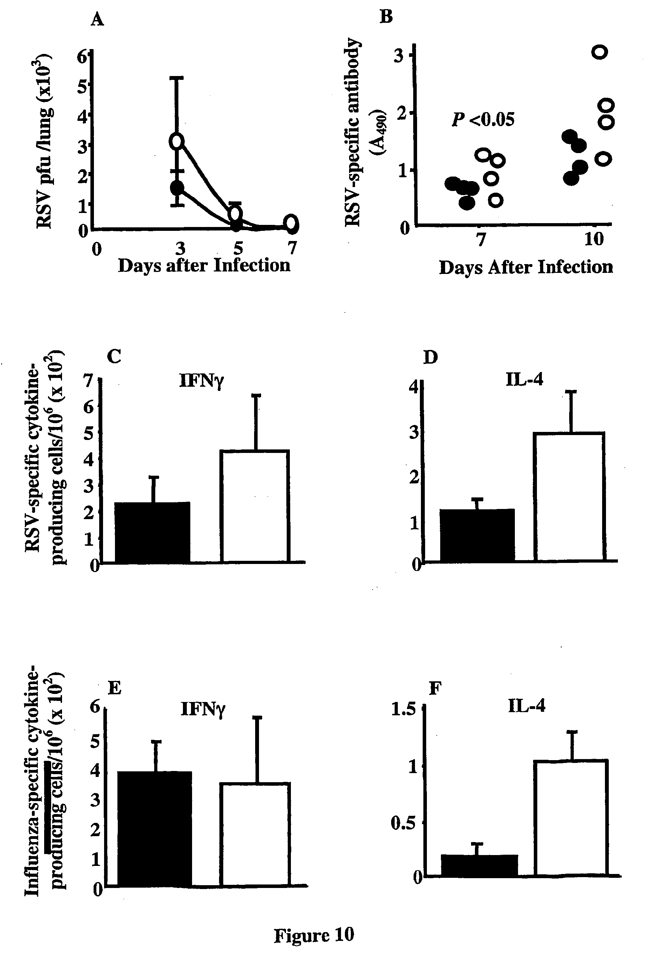

Figure 10: OX40:Ig enhanced virus specific antibodies and Th2 cytokines but reduced IFNγ in RSV infected mice treated with OX40:Ig. BALB/c mice were infected with RSV, treated with or without OX40:Ig fusion protein and 7 days later serum and lung cells were removed. RSV titre in the lung was determined by plaque assay of homogenates from control (open circles) or OX40:Ig treated (closed circles) mice (a). RSV specific antibody in the serum of control (■) or OX40:Ig treated (□) mice was determined by ELISA; each point representing an individual mouse (b). The frequency of lung cells secreting IFNγ (c and e) and IL-4 (d and f) in lung cell suspensions from RSV (c and d) or influenza virus (e and f) infected mice was determined by ELISPOT assay. The results represent the mean and standard deviation of 5 mice per group. Open and filled bars represent OX40:Ig and control treated mice, respectively;

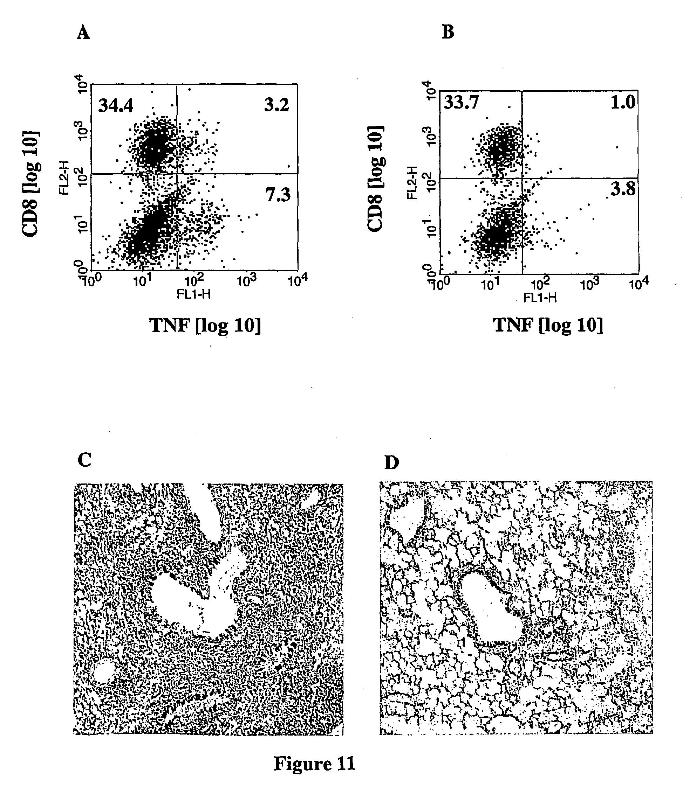

Figure 11: OX40:Ig treatment reduced TNF and cellular infiltration into the lung. Influenza virus infected mice were treated with PBS (a and c) or OX40:Ig (b and d). 7 days later lung cells were removed and stained for CD8 and intracellular TNF. Figures a and b show representative dot plots of airway CD8⁺T cells (y axis) versus TNF (x axis). In some experiments the lungs from PBS (c) or OX40:Ig (d) treated mice were inflated and fixed with 2% formaldehyde, paraffin embedded and sections stained with H and E. These sections are representative of 5 mice per group in 3 independent experiments;

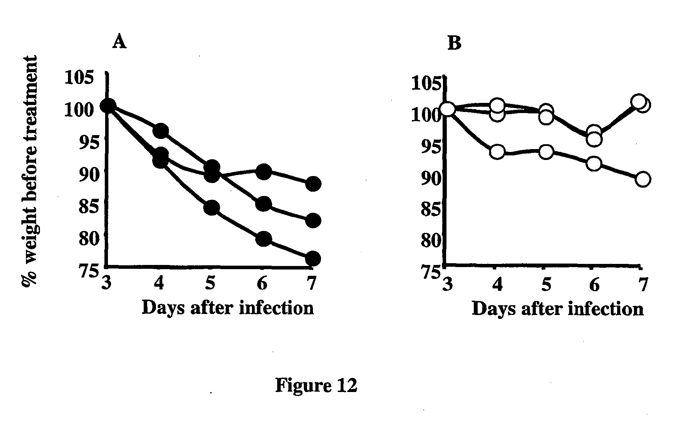

Figure 12: Delayed OX40:Ig treatment reversed influenza virus induced weight loss. Mice (n=5) were infected with influenza virus (X31) on day zero and treated with PBS (■) or OX40:Ig (□) on days 3, 5 and 6 of infection. Percent weight loss is calculated from the day of treatment, each line representing and individual mouse.

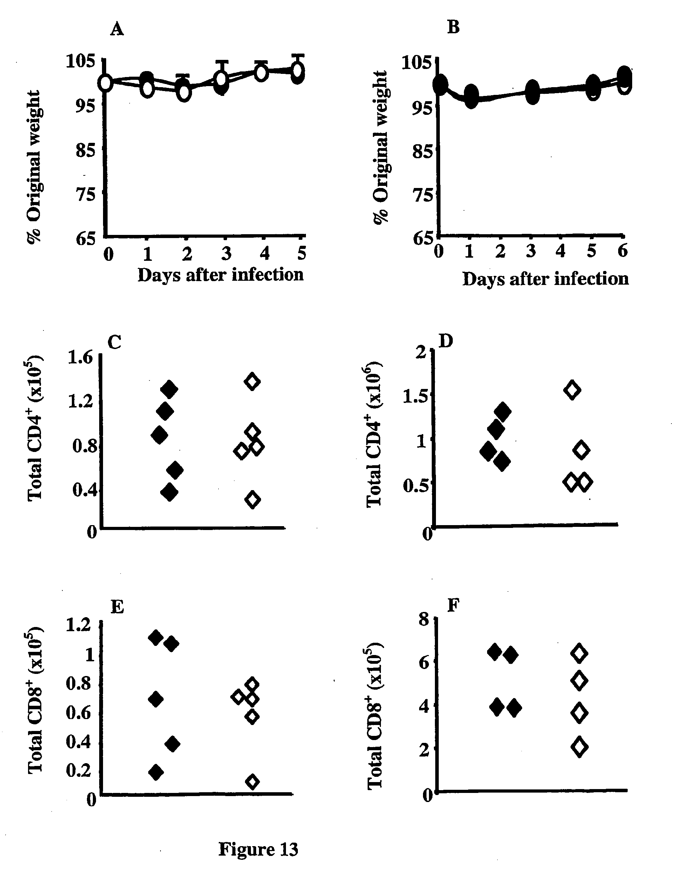

Figure 13: OX40:Ig did not alter recall responses to influenza and RSV following re-challenge. BALB/c mice (n=4) were infected with either influenza virus (a, c and e) or RSV (b, d and f) and treated with PBS (■) or OX40:Ig (□). 3 weeks later, mice were challenged with homologous virus and weight loss (a and b), CD4⁺ T cell (c and d) and CD8⁺ T cell (e and f) infiltrate 5 or 6 days post-infection was measured;

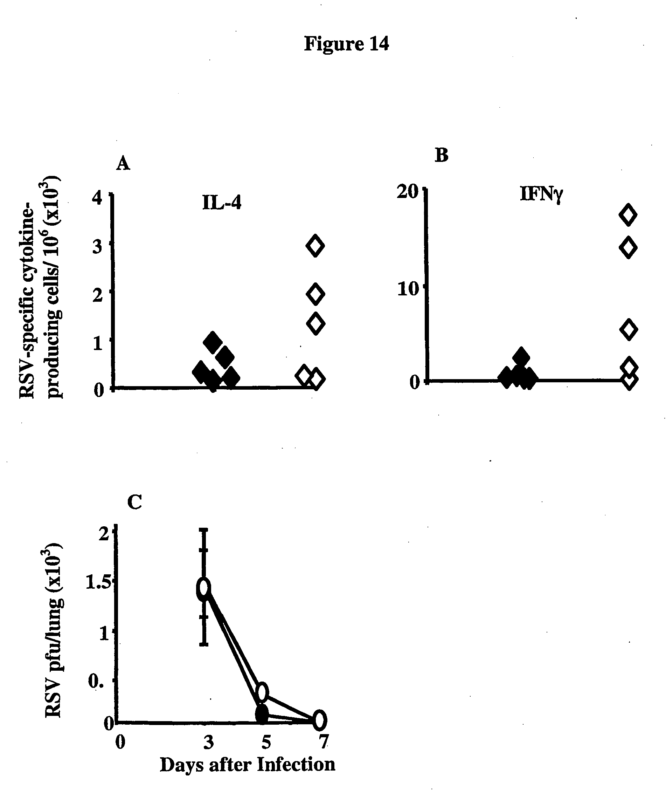

Figure 14: OX40L:Ig treatment did not enhance RSV induced illness and promoted virus-specific IFNγ and IL-4 responses. Mice were infected with RSV and treated with OX40L:Ig. The frequency of RSV specific lung T cells producing IL-4 (a) and IFNγ (b) was determined by ELISPOT. The results represent individual control (■) or OX40L:Ig (□) treated mice. RSV titres were determined in lung homogenates from control (closed symbols) or OX40L:Ig (open symbols) treated mice (c);

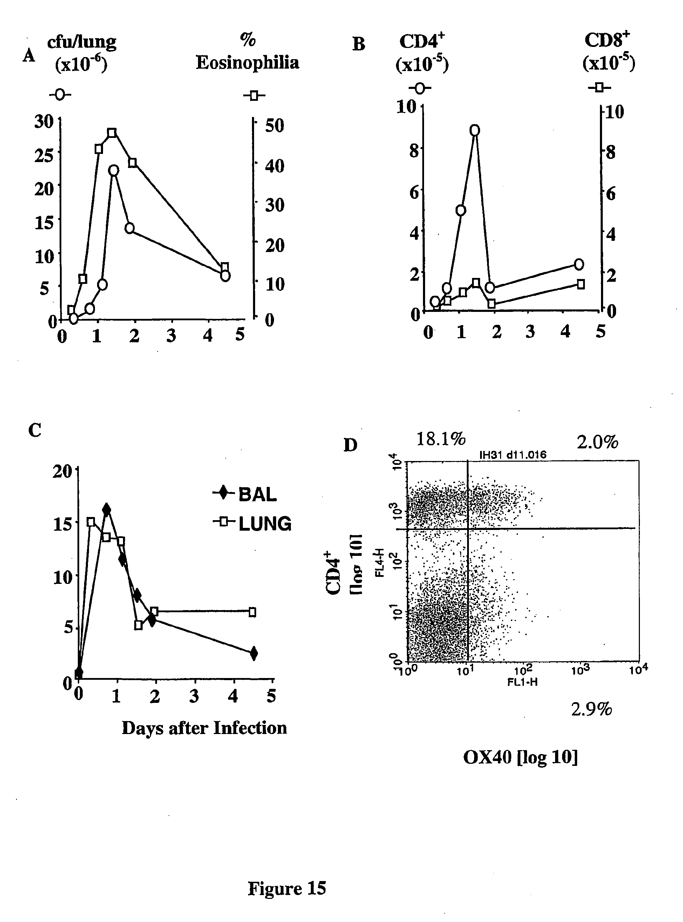

Figure 15: Time-course of *C.neoformans* infection is accompanied by OX40 expression by CD4⁺ T cells in the lung and airways.
   The number of cfu/lung was determined by plating out lung homogenate in 10-fold serial dilutions on sabouraud dextrose, and BAL eosinophils were enumerated as granulocytes by flow cytometry and confirmed using H and E stained cytocentrifuge preparations over a 45-day time-course experiment (a). Cells from the lungs of *C. neoformans* infected mice taken on days 0,2,7,11,14,19 and 45 days post-infection were stained with anti-CD4 and anti-CD8 antibody and analysed by flow cytometry. Total numbers were determined by multiplying the percent positive cells by flow cytometry with the total viable cells recovered from the lung (b). OX40 expression by CD4⁺ T cells in the BAL and lung was analysed by flow cytometry (c). A representative plot of CD4 (y axis) versus OX40 (x axis) is shown in panel d and is representative of 4-5 mice per group in two individual experiments;

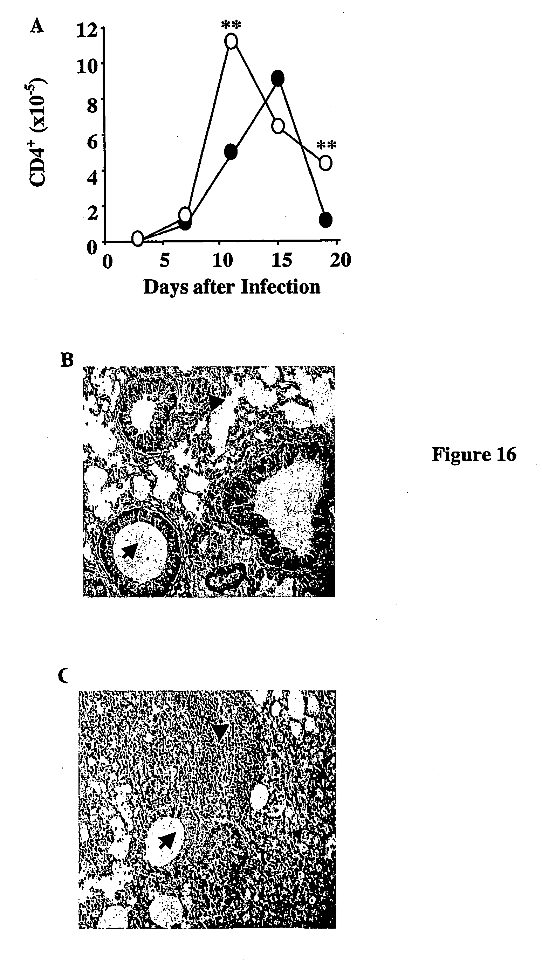

Figure 16: OX40L:Ig enhances CD4⁺ T cell numbers in the lung during *C.neoformans* infection.
   C57BL/6 mice (n=4) were infected with *C. neoformans* and treated with control Ig (●) or OX40L:Ig (○) on days 0, 2, 5, 8, and 11 after infection. Lung cells were taken on days 0,2,7,11,14, and 19 post-infection and stained with anti-CD4 antibody. Total numbers were determined by multiplying the percent positive cells by flow cytometry with the total viable cells recovered from the lung (a). **, *p* < 0.005 for control Ig vs OX40L:Ig. 11 days after infection, lungs from mice treated with either control Ig (b) or OX40L:Ig (c) were inflation fixed with 10% formaldehyde, paraffin embedded and sections stained with H and E. These sections are representative of 5 mice per group in 3 independent experiments;

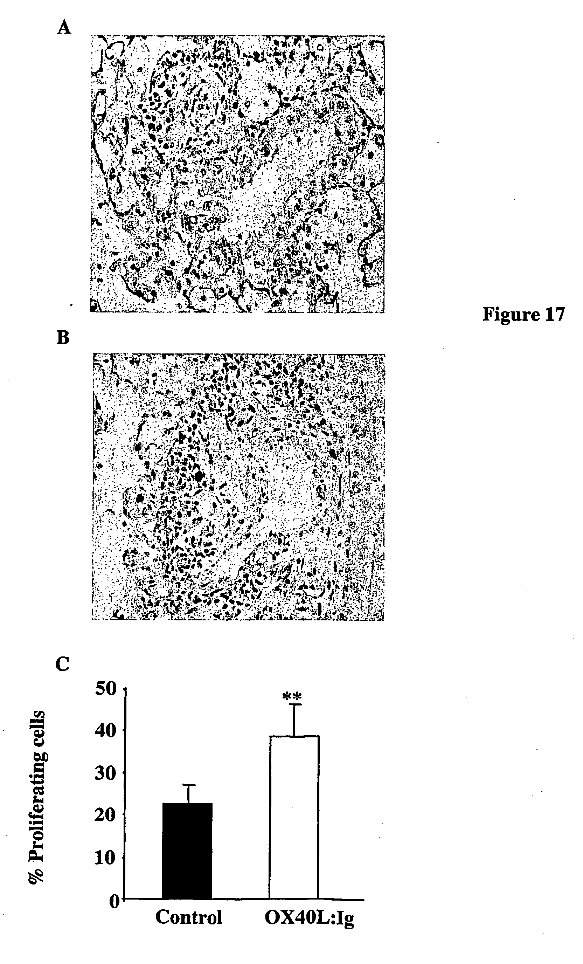

Figure 17: OX40L:Ig enhances cellular proliferation in the lung
   Cell proliferation in the lungs of *C.neoformans* infected mice treated with isotype control Ig (a) and OX40L:Ig (b) was analysed by injection of BrdU 90 minutes before sacrifice and stained with anti-BrdU monoclonal antibody. Proliferation of cells in the lungs was obtained by counting proportion of positively stained within perivascular infiltrates with at least 500 cells counted per section (c);

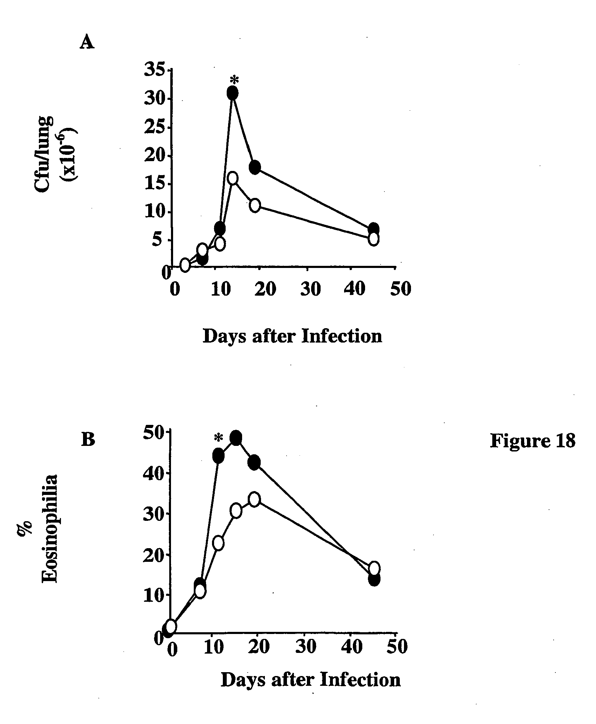

Figure 18: OX40L:Ig administration increases pathogen clearance and reduces associated pulmonary eosinophilia during *C.neoformans* infection.
   *C.neoformans* infected mice were treated with either control Ig (●) or OX40L:Ig (○) days 0,2,5,8,11 and 14 days post-infection. At multiple time-points (days 0,2,7,11,14,19), cfu/lung was determined from lung homogenate (a) and BAL eosinophils were enumerated in H and E stained cytocentrifuge preparations (b). *, *p* < 0.05 and **, *p* < 0.005 for control Ig vs OX40L:Ig;

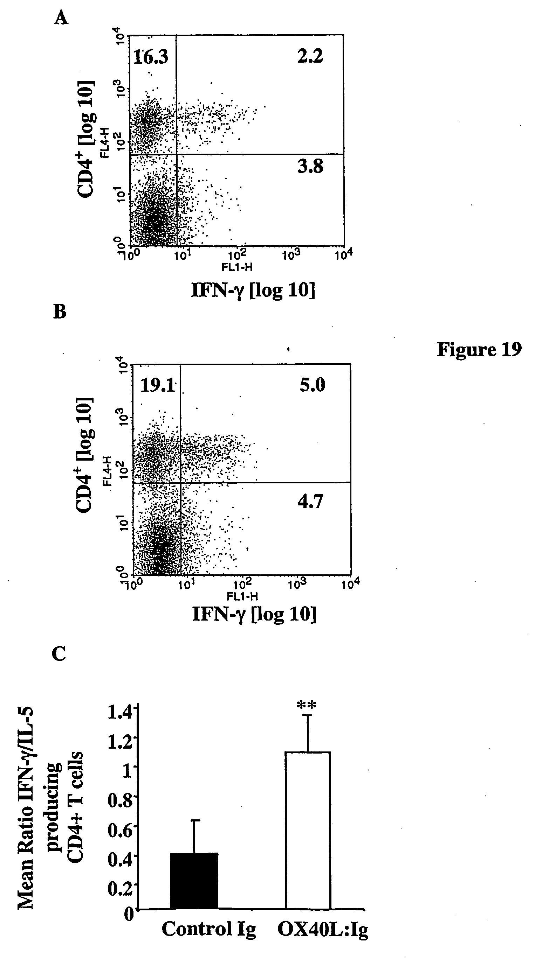

Figure 19: Enhanced OX40 signalling promotes IFN-γ production by CD4⁺ T cells in the lung during *C.neoformans* infection.
   IFN-γ production by CD4⁺ T cells 12 days post-infection was evaluated by intracellular cytokine staining in Cryptococcus infected control (a) and OX40L:Ig (b) treated mice. These results are representative of three experiments containing 5 mice per group. The ratio of IFN-γ/IL-5 producing CD4⁺ T cells was calculated by dividing percentage of IFN-γ-expressing CD4⁺ T cells by the percentage producing IL-5, as detected by intracellular cytokine staining (c). **, *p* < 0.005 for control Ig vs OX40L:Ig; and

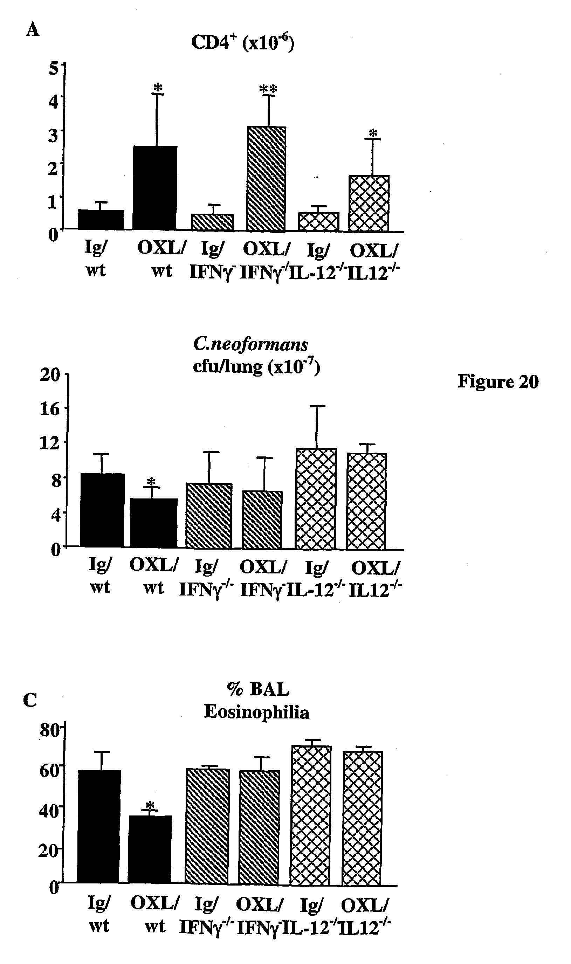

Figure 20: IFN-γ and IL-12 mediate OX40L:Ig-dependent inhibition of eosinophilia and promotion of *C.neoformans* clearance but not CD4⁺ T cell activation.
   Groups of 5 wild type (C57BL/6), IFNγ^{-/-} and IL12^{-/-} mice (C57BL/6 background) were infected with *C.neoformans* and treated with either OX40L:Ig or control antibody on days 0,2,5,8. Mice were sacrificed 12 days post infection. CD4⁺ T cells (a) in the lung of infected mice were determined by multiplying the percent positive cells by flow cytometry with the total viable cells recovered from the lung. Eosinophils were enumerated from H and E stained cytocentrifuge preparations of BAL fluid (b). The cfu/lung were measured from lung homogenate (c). All results represent mean values of 5 mice per group. *, *p* < 0.05 and **, *p* < 0.005 for control Ig vs OX40L:Ig in either wild type or IFN-γ^{-/-} mice.

### Examples

### Example 1

Mice were infected separately with influenza virus, with RSV, or with a control infectious agent, *Cryptococcus neoformans,* which is an encapsulated yeast that causes lung eosinophilia in immuno-compromised patients. Unlike the viral infections, it is the replication of Cryptococcus that causes illness.

One group of mice was treated with OX40:Ig (which can bind the OX40L ligand naturally expressed on antigen presenting cells and prevent T cell co-stimulation); the control group was treated with phosphate-buffered saline (PBS). Treatment was performed on the day of infection and then on alternate days until the experiment was terminated and the animals killed. Weight loss and appearance were monitored throughout. At sacrifice, lung lavage, residual lung tissue, mediastinal lymph nodes and serum were removed.

### Influenza virus infection:

### Weights

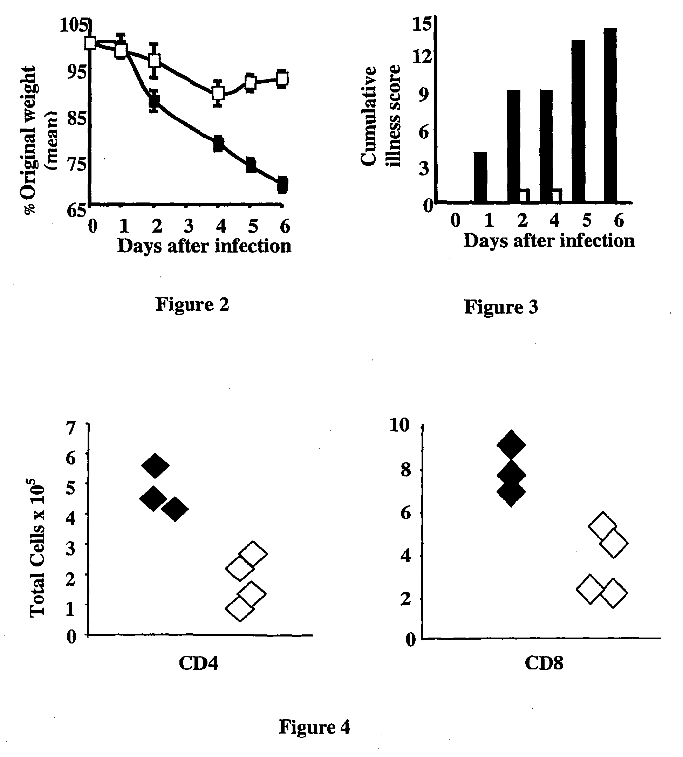

Figure 2 shows that the weight loss induced by influenza is inhibited in mice treated with OX40:Ig (open symbols) compared with PBS-treated mice (closed symbols).

### Illness severity

Figure 3 illustrates that a remarkable beneficial effect of OX40:Ig administration was also shown when the illness was scored using an established grading system based on degree of cachexia and mobility. OX40:Ig (open bars) treatment almost eradicates influenza-induced illness seen in control animals (closed bars).

### Cellular Infiltration

Figure 4 illustrates that OX40:Ig treatment (open symbols) inhibited cellular infiltration into the bronchoalveolar lavage (BAL) during influenza infection. A reduction in total cell numbers and total lymphocytes (data not shown), together with total CD4⁺ and CD8⁺ T cells was observed in treated groups compared with control animals (closed symbols). A reduction in TNF-producing CD8⁺ T cells in the lung was also observed following OX40:Ig administration. TNF has been implicated as a causative agent in viral-induced weight loss: therefore reduced levels of this in OX40:Ig-treated animals explains inhibition of weight loss.

### RSV infection:

### Weight Loss and Illness

In comparison to influenza, respiratory syncytial virus (RSV) is a relatively mild respiratory infection. BALB/c mice experience mild weight loss and illness during the first 6-8 days of infection.

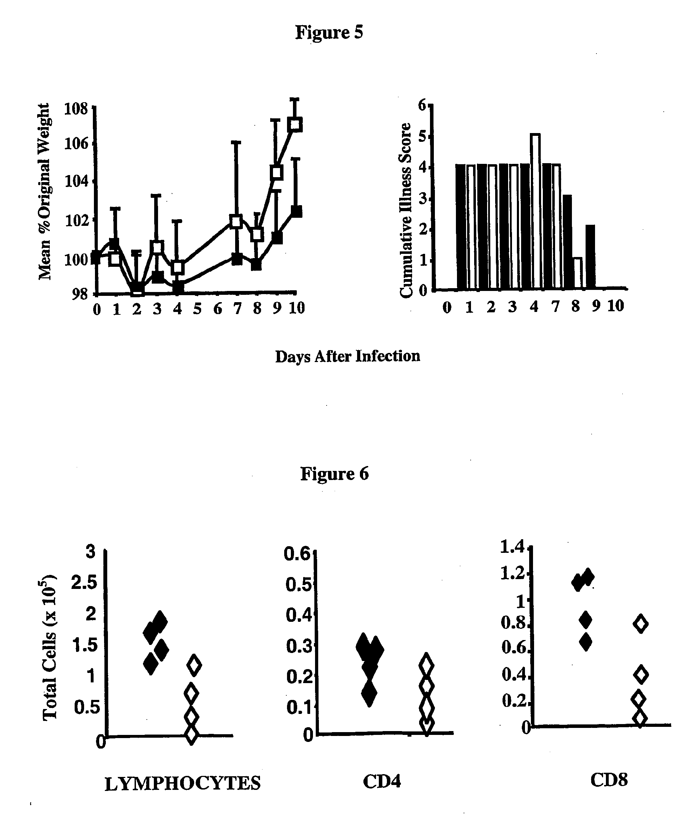

Figure 5 illustrates how OX40:Ig treatment reduced both weight loss and illness severity at later time-points (see below), although due to the mild nature of RSV infections, major differences between control animals (closed symbols) and OX40:Ig-treated mice (open symbols) were less than those during influenza infection.

### Cellular Infiltration

Figure 6 illustrates that, as observed in influenza-induced immunopathology, OX40:Ig treatment (open symbols) reduced cellular infiltration of lymphocytes, CD4⁺ and CD8⁺ T cells into the BAL during a primary RSV infection.

### Antibody Production

RSV also induces protective humoral immune responses during infection. Most important for vaccine development is an observation that OX40:Ig administration has led to an increase in total anti-RSV IgG levels in the serum of RSV-infected mice (data not shown).

Accordingly, OX40:IgG fusion proteins and biologies with similar binding activity are provided (e.g. in dosages as disclosed above), as vaccine adjuvants for vaccines against viruses, for example against influenza or RSV or related viruses (e.g. negative-stranded viruses with segmented genomes: for which see e.g. WO 91/03552). The adjuvants can be given either at the same time as or after the vaccines themselves. Vaccines against viruses, such as against influenza or RSV or related viruses, with which the adjuvants can be used in combination or association, include conventional vaccines directed against such viruses. Generally the range of vaccines with which the adjuvants can be associated or combined includes killed vaccines, subunit vaccines and genetically disabled vaccines as for example those disclosed in relation to influenza viruses and related viruses in WO 91/03552.

### Cryptococcus neoformans infection (comparison)

In a murine model using BL/6 mice, *Cryptococcus neoformans* induces a large eosinophilic infiltration into the lung. OX40:Ig treatment did not affect these levels. During a primary *Cryptococcus* infection, mice experience little or no weight loss. Therefore, extent of weight increase was measured. OX40:Ig treatment actually appeared to induce slight weight loss compared with control mice which gained weight.

Cellular infiltration into the BAL during infection was reduced, although the extent of this varied between experiments. OX40:Ig treatment was also shown not to significantly affect clearance of *Cryptococcus* from the lung, spleen or brain compared with control mice.

Respiratory syncytial and influenza viruses cause acute respiratory infections and are cleared with relative ease by the immune system. Yet, the immunopathological results of these infections can be very serious. The disclosure above indicates that inhibition of OX40-mediated T cell costimulation can reduce these inflammatory responses. OX40:Ig treatment can also induce an increased anti-RSV antibody response, probably due to OX40:Ig binding to OX40L on B cells, inducing IgG secretion (although the inventors do not wish to be bound by this hypothesis).

Therefore, these results support that OX40:Ig has therapeutic usefulness in the treatment of viral-induced inflammatory responses and in promotion of protective antibody responses against a viral pathogen.

### Example 2

### Methods

### Mice and pathogens

8-12 week old female BALB/c and C57BL/6 mice (Harlan Olac Ltd, Bicester, UK) were kept in pathogen-free conditions according to Home Office guidelines. Influenza A strain X31 (hemagglutinin [HA] titre 1024) was a kind gift from Dr Alan Douglas (National Institute for Medical Research, London, UK). RSV (A2 strain) was grown in HEp-2 cells and assayed for infectivity as described is Bangham, et al, J. Virol. 56, 55-59 (1985). *C. neoformans* strain 52, was obtained from the American Type Culture Collection (Rockville, USA) and for infection grown to stationary phase (48-72 hours) at room temperature on a shaker in Sabouraud dextrose broth (1% neopeptone and 2% dextrose; Difco, Detroit, MI). The cultures were washed in saline, counted on a haemocytometer and diluted in sterile nonpyrogenic saline to the required infective dose.

### Production of OX40 and OX40 ligand fusion proteins

Murine OX40:mIgG1 fusion protein (OX40:Ig) was constructed by using a chimeric cDNA that contained the extracellular domain of OX40 fused to the constant region of murine IgG1. This construct was used to transfect clonal chinese hamster ovary cells and fusion protein was purified from the culture supernatant using protein G sepharose. The murine OX40L:murine IgG1 fusion protein (OX40L:Ig) was constructed as described in Weinberg, et al. J.Immunol. 164, 2160-2169 (2000) using a chimeric cDNA containing the C-terminal region of OX40L fused to the constant region of murine IgG1.

### Mouse infections and treatment

On day 0 BALB/c mice were anaesthetised and intranasally infected with 1 x 10⁶ pfu RSV or 50 HA units influenza virus (in 50 µl). *C. neoformans* infections (2 x 10⁴ pfu/mouse) were performed in C57BL/6 mice. Some groups of mice were injected intraperitoneally (i.p.) with 100 µg OX40:Ig or OX40L:Ig fusion proteins (Xenova pharmaceuticals, Cambridge, UK) on various days after infection as indicated in the text. The weight of mice and illness severity was monitored daily. Mice were sacrificed on days 6 or 7 after viral infection and on day 12 after *C. neoformans* infection by injection of 3 mg pentobarbitone and exsanguinated via the femoral vessels. Bronchoalveolar lavage (BAL), lung and serum were removed using methods described in Hussell, et al, J.Gen.Virol. 77, 2447-2455 (1996).

### Quantification of illness severity

Illness severity was scored daily using the following criteria: 0 = healthy, 1=barely ruffled fur, 2 =ruffled fur but active, 3 = ruffled fur and inactive, 4 = ruffled fur, inactive and hunched, 5 = dead.

### Cell recovery

BAL, lung tissue and serum were harvested by methods described in Hussell, et al. J.Gen.Virol. 77, 2447-2455 (1996). Briefly, the lungs of each mouse were inflated 6 times with 1ml 1mM EDTA in EMEM and placed in sterile tubes on ice. 100 µl BAL fluid from each mouse was cytocentrifuged onto glass slides. The remainder was centrifuged and the supernatant removed and stored at -70 °C in 200 µl aliquots for analysis of cytokines by ELISA. Cell viability was assessed using trypan blue exclusion and the pellet resuspended in RPMI containing 10 % FCS, 2 mM/ml L-glutamine, 50 U/ml penicillin and 50 µg/ml streptomycin (R10F) at a final concentration of 10⁶ cells/ml. Eosinophils were enumerated as granulocytes by flow cytometry, using forward and side scatter. Identification was confirmed by counting eosinophils in H and E stained cytocentrifuge preparations.

### Flow cytometric analysis of intracellular and cell surface antigens

1 x 10⁶ BAL and lung derived cells were stained using Cy-chrome^{™}-conjugated anti-CD4, anti-CD8-PE and either anti-CD45RB-FITC (all BD Pharmingen, Heidelberg, Germany) or anti-OX40-FITC (Serotec, Oxford, UK) for 30 min on ice. Cells were then fixed for 20 min at room temperature with 2 % formaldehyde. To detect intracellular cytokines, 10⁶ cells/ml were incubated with 50 ng/ml PMA (Sigma-Aldric, Poole, Dorset), 500 ng/ml ionomycin (Calbiochem, Nottingham, UK), and 10mg/ml brefeldin A (Sigma-Aldrich) for 4 hours at 37°C. Cells were then stained with either anti-CD4-Cy-chrome^{™} or anti-CD8-Cy-chrome^{™} and fixed as described above. After permeabilization with PBS containing 1 % saponin/1 % BSA/0.05 % azide (saponin buffer) for 10 min either FITC-conjugated anti-TNF or anti-IFNγ (Pharmingen) and PE-conjugated anti-IL4 (Pharmingen) diluted 1:50 in saponin buffer were added. 30 minutes later cells were washed once in saponin buffer and once in PBS/1 %BSA/0.1 % azide. All data was acquired on a FACSCalibur and analysed with CellQuest Pro software (BD Biosciences, Belgium).

### Lung histology

In some studies, lungs were inflated and fixed with 2 % formalin in PBS, 6-7 days after intranasal RSV or influenza virus and 12 days after *C. neoformans* infection. The inflated lungs were excised and embedded in paraffin wax by the histopathology department at the Hammersmith Hospital, UK. Thin sections were stained with haematoxylin and eosin (H and E). The lungs from 4-5 mice per group were analysed.

### Lung RSV titer

Clearance of RSV was assessed in lung homogenates 2 and 4 days after virus challenge. After centrifugation at 4000 rpm for 4 minutes, supernatant was titrated in doubling dilutions on HEp-2 cell monolayers in flat bottomed 96 well plates. Twenty-four hours later, monolayers were washed and incubated with peroxidase conjugated goat anti-RSV antibody (Biogenesis, Poole, UK). Infected cells were detected using 3-amino-9-ethylcarbazole (AEC), infectious units being enumerated by light microscopy.

### Lung influenza virus titer

The titer of influenza virus in the lungs was determined by hemagglutination assay. Briefly, lungs were removed, homogenized and centrifuged to remove large cell debris. 50 µl supernatant was incubated with 1% turkey red blood cells and the hemagglutination titer determined from the reciprocal of the highest dilution of virus showing agglutination (which represents 1 HAU/50 µl homogenate).

### Enumeration of C. neoformans from lung homogenates

Lungs were homogenised by passage through 100 µm cell strainers (BD labware, New Jersey, USA). 100 µl of cell suspension was diluted in PBS and incubated at room temperature for 48 hours on sabouraud dextrose agar plates (Sigma). The total colony forming units per lung was then determined (number of colonies x dilution factor x original cell suspension volume).

### RSV-specific antibody ELISA

Serum antibody was assessed by ELISA as described in Hussell, et al. Eur J Immunol 27, 3341-3349 (1997); Walzl, et al,. J.Exp.Med 193, 785-792 (2001). ELISA antigen was prepared by infecting HEp-2 cells with RSV strain A2 at 1 pfu/cell. When a significant cytopathic effect was observed the infected cells were harvested, centrifuged at 400 g, resuspended in 3 ml distilled water and then subjected to 2 minutes of sonication (Ultrawave Ltd, Cardiff). 50 µl aliquots were stored at -20 °C until required. Microtitre plates were coated overnight with 100 µl of a 1/200 dilution of either sonicated RSV or HEp-2 cells alone. After blocking with 2 % normal rabbit serum for 2 hours, dilutions of test samples (diluted in PBS containing 1 % HEp-2 lysate) were added for a further hour at room temperature. Bound antibody was detected using peroxidase-conjugated rabbit anti-mouse Ig and *O*-phenylene-diamine (Sigma) as a substrate. The reaction was stopped with 50 µl 2.5 M sulphuric acid. Optical densities were read at 490 nm. The amount of RSV specific antibody was determined by subtracting the optical density obtained by incubating serum on RSV coated plates from the same sample incubated on HEp-2 coated plates.

### Enzyme-linked immunospot (ELISPOT) for murine cytokines

ELISPOT assays were performed as described in Hussell, & Openshaw, J.Immunol. 165, 7109-7115 (2000). Sterile filter plates (multiscreen filter plates, Millipore corporation, Bedford MA, USA) were coated overnight at 4 °C with 40 ug/ml rat anti-mouse IL-4, IL-5 or IFNγ (Pharmingen), washed and then blocked for 2 hours with 10 % FCS/RPMI. Lung cells were added at 1 x 10⁶ cells/well with 4 doubling dilutions and cultured for 48 hours with either 1 pfu/cell RSV, 0.1 HA/cell influenza virus X31, 5 x 10⁴ heat inactivated Cryptococcus or medium alone. After removal of cells the site of cytokine production was detected using 1 µg/ml biotinylated rat anti-mouse IL-4, IL-5 or IFNγ followed by alkaline phosphatase-labelled streptavidin using BCIP/NBT as the substrate. The frequency of cytokine producing cells was enumerated by light microscopy.

### Statistics

Statistical significance was evaluated using the student t test, 2 tailed assuming unequal variance within the Minitab software program.

### Results

### Expression of OX40 on CD4⁺ and CD8⁺ T cells during lung infection

Expression of OX40 on activated CD4⁺ T cells has previously been described but expression by CD8⁺ T cells is unclear. Cells from the lungs of mice infected with influenza virus, RSV or *C. neoformans* were analysed for CD4, CD8 and OX40 expression using flow cytometry. Only low levels of OX40 were observed on lung cells from uninfected mice. After infection with all pathogens however, OX40 expression increased significantly on CD4⁺ T cells (Figure 7a, c and e). The level of expression was higher following influenza infection than in *C. neoformans* or RSV-infected mice. CD8⁺ T cells recruited in response to influenza infection also expressed high levels of OX40 (Figure 7b). Significant but low levels of OX40 were also observed on CD8⁺ T cells following RSV but not *C. neoformans* infection (Figure 7d, e).

### OX40L: Ig promotes IFNγ production and prevents Cryptococcus neoformans induced lung eosinophilic

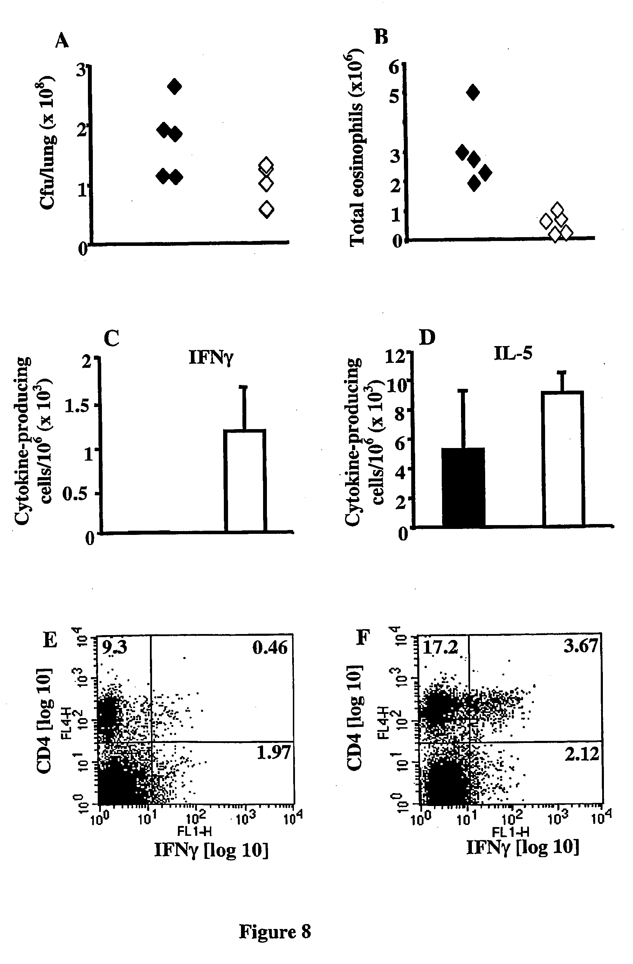

Intranasal *C. neoformans* infection of C57BL/6 mice induces a non-protective Th2 cytokine response resulting in the accumulation of 50-80% eosinophils in the lung. The OX40L:Ig binds to OX40 on the T cell and mimics APC derived co-stimulatory signals. *C. neoformans* could still be recovered from the lung of mock treated C57BL/6 mice 12 days after infection. Treatment with OX40L:Ig significantly reduced both the colony forming units recovered (Figure 8a) and lung eosinophilia (Figure 8b).

This alteration in the pathological phenotype was accompanied by an increase in the frequency of cells producing IFNγ (Figure 8c) whereas the frequency of IL-5 producing cells was similar after OX40L:Ig treatment (Figure 8d). Intracellular cytokine staining revealed that both CD4⁺ (Figures 2 e and f, p 0.019) and CD8⁺ (data not shown) T cells were the cellular source of IFNγ. Treatment with OX40L:Ig therefore enhanced IFNγ production by T cells, reduced lung eosinophilia and promoted clearance of *C. neoformans* from the lung. The converse treatment with OX40:Ig also reduced lung eosinophilia (from 1.57 x 10⁶ +/- SD 0.44 to 0.99 x 10⁶ +/-SD 0.3; p0.007) but not the pathogen burden. A reduction of CD⁴⁺ T cell numbers in the BAL (from 9.1 x 10⁴ +/- SD 0.95 to 5.0 x 10⁴ +/- SD 2.4; p0.02) was also observed. In the case of OX40:Ig treatment eosinophilic responses were abrogated because CD4⁺ T cells numbers were reduced.

### OX40:Ig treatment prevented RSV and influenza virus induced illness and immunopathology.

Lung infection of naïve BALB/c mice with RSV or influenza virus results in TNF and IFNγ driven weight loss and cachexia and is accompanied by the recruitment of CD4⁺ and CD8⁺ T cells secreting type 1 cytokines. In this situation it is therefore desirable to reduce the type 1 cytokine phenotype. RSV and influenza infected mice were treated with OX40:Ig. In influenza infected, control treated mice the experiment had to be terminated 6 days after infection due to severe weight loss. In a parallel group however OX40:Ig treatment abolished the weight loss (Figure 9a) and illness severity. PBS treated influenza infected mice, especially at day 6, appeared hunched, immobile and severely cachexic; whereas OX40:Ig treated mice were indistinguishable from uninfected controls (Figure 9b). A similar beneficial effect was also observed following RSV infection (data not shown). During both infections OX40:Ig treatment significantly reduced the total cell recruitment, neutrophils, CD4⁺ (Figure 9c and d) and CD8⁺ (Figure 9e and f) T cells in the BAL. Mediastinal lymph nodes were also reduced in size in OX40:Ig treated mice (from 14.0 x 10⁵ +/- 3.4 to 8.3 x 10⁵ +/- SD 2.6, p 0.04).

The clearance of influenza and RSV (Figure 10a) was not affected by OX40:Ig treatment (data not shown). Though RSV titres were higher in some OX40:Ig treated mice this did not reach significance (p>0.05) and the kinetics of RSV clearance was identical with PBS treated controls. All mice had cleared the virus by day 7. This may be due to the fact that innate immune mechanisms are very effective against lung viral infections. During influenza infection in the BAL there were 1.07 x 10⁴ +/- SD 0.8 NK cells in mock treated mice compared to 0.9 x 10⁴ +/- 0.3 in OX40:Ig treated mice. Similarly in the lung there were 2.6 x 10⁴ +/- SD 0.2 and 2.4 +/- SD 0.4 NK cells in mock and OX40:Ig treated mice, respectively. In addition virus specific antibody titres were elevated in OX40:Ig treated mice (Figure 10b), which may be linked to the alteration in Th2 cytokines. Though there was no difference in the frequency of cells producing IFNγ (Figure 10 c and e) a significant increase was observed for IL-4 (Figure 10 d and f) and IL-5 (data not shown) in OX40:Ig treated RSV and influenza infected mice. By intracellular cytokine staining we also observed a decrease in the proportion of BAL CD8⁺ and CD8⁻ T cells producing TNF in OX40:Ig treated mice (figure 11a and b). The absolute number of TNF-producing CD8⁺ T cells was also significantly reduced in OX40:Ig treated mice. We have previously shown that TNF is intimately associated with illness and weight loss in both of these infections (Hussell, et al Eur J Immunol 31, 2566-2573 (2001)). The reduction in TNF by OX40:Ig treatment may therefore explain the beneficial effects observed in the current study. Treatment with anti-TNF antibodies also reduces total inflammatory infiltrate to the lung (Hussell, et al Eur J Immunol 31, 2566-2573 (2001)); an effect clearly evident in H and E stained sections of lung tissue from OX40:Ig treated influenza infected mice. Cellular infiltration around the airways and blood vessels was markedly reduced in OX40:Ig treated mice (Figures 11c and d). *Delayed OX40:Ig treatment reduced the immunopathology in influenza infected mice with established illness.*
For OX40:Ig treatment to be used in a clinical setting it must be effective in patients with established disease. We therefore infected BALB/c mice with influenza virus and administered OX40:Ig when they had lost 20% of their starting weight. Figure 12 shows that delayed treatment reversed a decline in weight.

### Recall responses to secondary infections were not altered by OX40:Ig treatment.

Since cellular recruitment was significantly reduced by OX40:Ig treatment during primary viral infections, we determined whether recall responses were also affected by challenging mice with homologous virus 3 weeks after the first infection. Influenza virus infected mice displayed less weight loss during a secondary challenge compared to a primary infection (compare weight loss profiles in figures 3a and 7a). Treatment with OX40:Ig during the first infection did not alter the milder weight loss during re-challenge with either influenza (Figure 13a) or RSV (Figure 13b). Similarly CD4⁺ and CD8⁺ T cell recruitment was unaltered in re-challenged mice following influenza (figure 13 c and e) or RSV (figure 13 d and f) secondary infections.

### OX40L:Ig administration during RSV enhanced RSV-specific IFNγ production.

The role of OX40 signalling in responses to RSV was further investigated by administering OX40L:Ig to infected mice during lung infection (i.e. the converse treatment to OX40:Ig treatment). Surprisingly, weight loss was not affected (data not shown) despite enhanced levels of RSV-specific IFNγ and IL-4 in 3/5 mice (Figure 14). Clearance of RSV was identical to control treated mice (Figure 14c). It may therefore be possible to promote anti-viral immunity to RSV using OX40L:Ig without enhancing illness.

### Discussion

We show that 1) OX40 is up-regulated on both CD4⁺ and CD8⁺ T cells after lung infection, 2) expression occurs in both Th1 and Th2 cytokine environments and, 3) manipulation of this single late co-stimulatory signal alleviates the symptoms of three clinically important lung infections. The effect on immunopathology is associated with a change in the balance of Th1 and Th2 cytokines. OX40L:Ig administration enhanced IFNγ production in both *C. neoformans* and RSV infections: in the case of *C. neoformans,* reducing harmful Th2-mediated pulmonary eosinophilia. Administration of OX40:Ig during *C. neoformans* infection also reduced eosinophilia by antagonizing CD4⁺ T cell numbers in the lung. The beneficial effect of OX40:Ig administration is not restricted to Th2 induced immunopathology since Th1-mediated illness, weight loss and lung inflammatory cell infiltration during RSV or influenza virus infection is also abrogated. Although OX40 expression by activated CD4⁺ T cells has previously been described, few have reported expression by CD8⁺ T cells (Al-Shamkhani, et al. Eur.J.Immunol. 26, 1695-1699 (1996)). Furthermore, the up-regulation of OX40 on CD8⁺ T cells is more dramatic during influenza compared to RSV infection but the significance of this is not known.

The use of fusion proteins provides a novel approach to study co-stimulatory interactions *in vivo* compared with inhibitory anti-OX40 antibodies, which though prevent T cell co-stimulation, also block the signal to the APC via OX40L. With antibody treatment it is therefore difficult to ascertain whether the observed effects are due to reduced T cell activation or altered APC function since ligation of OX40L has been shown to enhance maturation of human dendritic cells *in vitro* (Ohshima, et al, J.Immunol. 159, 3838-3848 (1997)) and impaired APC function has been reported in OX40L^{-/-} mice (Murata, et al. J.Exp.Med 191, 365-374 (2000)). Stimulation of OX40L on B cells also increases Ig secretion (Stuber et al. Immunity. 2, 507-521 (1995); Morimoto, et al J.Immunol. 164, 4097-4104 (2000)), an effect observed in our study following OX40:Ig administration during RSV infection. This disadvantage of losing the signal to the T cell and APC is also relevant in OX40 knockout mice, which have been used to study OX40 co-stimulation in asthma (Jember, et al J.Exp.Med. 193, 387-392 (2001) and LCMV infection Kopf, et al Immunity. 11, 699-708 (1999). The role of OX40 in non-immunological processes is not known and so knockout mice may suffer from other developmental problems.

Although OX40:Ig administration during RSV or influenza infection reduced lung T cell infiltration, the clearance of both viruses was remarkably unaffected. The increase in virus specific antibody may compensate for reduced cellular responses.

Alternatively, innate immune mechanisms, or the residual inflammatory infiltrate remaining in treated mice, may be enough to mediate virus clearance. Although NK cells play a pivotal role in immunity to RSV infection (Hussell, & Openshaw, J.Immunol. 165, 7109-7115 (2000)), little is known of their function during influenza virus infection. Increased susceptibility to influenza in the senescence-accelerated mouse is associated with impaired activity of NK cells, but CD8⁺ T cells are also defective in this model (Dong, et al, J Infect.Dis. 182,391-396 (2000)). NK cell responses in the current study were unaltered by OX40:Ig administration. RSV and particularly influenza virus are highly inflammatory in the mouse. It is possible that a moderate reduction in the vigour of the inflammatory response may be beneficial without affecting virus clearance. Our results are consistent with the efficient control of Theiler's murine encephalomyelitis virus, *Nippostrongylus brasiliensis,* Leishmania major (Pippig, et al J.Immunol. 163, 6520-6529 (1999)) and influenza virus (Kopf, et al. Immunity. 11, 699-708 (1999) in OX40 knockout mice.

An important consideration for therapeutic intervention with OX40:Ig is to determine if memory is affected. During re-challenge with RSV or influenza, weight loss, recruitment of T cells and the frequency of virus-specific cytokine producing cells were unaffected in OX40:Ig treated mice. It is important to note that both fusion proteins were administered during the *first* infection and that their effect may be different if administered during re-challenge.

The effect of OX40:Ig on virus induced illness and weight loss is striking. In immunocompetent mice over-exuberant T cell responses and elevated TNF levels induce rapid and severe illness (Hussell, & Openshaw, J.Immunol. 165, 7109-7115 (2000); Hussell, et al, Eur J Immunol 31, 2566-2573 (2001). OX40:Ig reduced both CD4⁺ and CD8⁺T cells and weight loss during lung virus infection, reiterating the immunopathological nature of the disease. Although a small (but significant) relative reduction of TNF producing CD8⁺ T cells was observed in OX40:Ig treated mice, this decrease is substantial when the actual numbers in the lung are calculated (mean control = 2.1 x 10⁴, mean OX40:Ig = 0.89 x 10⁴, p 0.01).

The reduction of both CD4⁺ and CD8⁺ T cells in OX40:Ig treated mice is consistent with the expression of OX40 on both cell subsets. This is in contrast to a previous study in influenza or LCMV infected OX40 knockout mice, where only the CD4⁺ T cells were affected ²³. The discrepancy may arise from differences between the mouse or virus strain used and/or the fact that our study used fusion proteins whereas Kopf *et al.* used OX40 knockout mice. Similarly, intracellular IFNγ in CD4⁺ but not CD8⁺ T cells was reduced in LCMV infected OX40 knockout mice but the same analysis was not performed for influenza virus. We show reduced intracellular IFNγ and TNF in both CD4⁺ and CD8⁺ T cells in mice treated with OX40:Ig, which is consistent with the decrease in IFNγ protein production previously demonstrated in OX40 knockout mice (Kopf, et al. Immunity. 11, 699-708 (1999)). It is likely that the T cell requirement for OX40 co-stimulation will alter with the infection model. Though CTL responses to LCMV do not require CD4⁺ T cell help we do not know if the same is true for influenza virus or RSV infection. Indeed, thymectomised mice reconstituted with influenza immune splenic T cells that have been depleted of CD4⁺ T cells, only generate half the normal level of cytotoxic T cells after a subsequent virus challenge (Lightman, et al Immunology 62, 139-144 (1987)).

The reduced cellular infiltration in virus-infected mice treated with OX40:Ig can be explained in several ways, although the inventors do not wish to be bound by theory. OX40 ligation on the T cell has been shown to enhance primary T cell expansion, retention and anti-apoptosis gene expression (Gramaglia, et al, J.Immunol. 165, 3043-3050 (2000); Gramaglia, et al, J.Immunol. 161, 6510-6517 (1998); Weinberg, et al, Semin.Immunol. 10, 471-480 (1998); Lightman, et al. Immunology 62, 139-144 (1987)) but reduce peripheral T cell deletion (Maxwell, et al. J.Immunol. 164,107-112 (2000)) and activation induced cell death (Weinberg, et al. Semin.Immunol. 10, 471-480 (1998)). OX40 ligand is also expressed by endothelial cells (Imura, et al. J.Exp.Med. 183, 2185-2195 (1996) and therefore OX40:Ig fusion protein treatment may block lymphocyte extravasation into the parenchyma. Transgenic mice expressing OX40 ligand under the control of the CD11c promoter have increased CD4⁺ T cells in B cell follicles (Brocker, et al. Eur.J.Immunol. 29, 1610-1616 (1999)). We show reduced cell recruitment to the mediastinal lymph nodes by OX40:Ig treatment, which could represent altered extravasation or impaired T cell priming. Though OX40:OX40L interactions influence T cell migration into the CNS during EAE (Nohara, et al. J.Immunol. 166,2108-2115 (2001)) no defects in T cell homing have been detected in OX40L knockout mice (Chen, et al. Immunity. 11, 689-698 (1999)).

Effective pulmonary clearance of *C. neoformans* requires T cell immunity (Huffnagle, et al. J.Exp.Med. 173, 793-800 (1991); Hill & Harmsen, J.Exp.Med. 173, 755-758 (1991)), monocytes and neutrophils (Mambula, et al. Infect.Immun. 68, 6257-6264 (2000)). Administration of OX40L:Ig enhances anti-tumour immunity *in vivo* by increasing the number and survival of tumour specific T cells. We showed that CD4⁺ and CD8⁺T cells increased in the lung of *C. neoformans* infected mice treated with OX40L:Ig. In OX40L:Ig treated mice enhanced IFNγ secreting T cells coincided with a reduction in fungal burden and eosinophilia in the lung. The converse treatment with OX40:Ig also reduced the number of CD4⁺ T cells in the lung and hence the level of Th2 cytokines essential for eosinophil recruitment and survival. A similar effect on eosinophils is also observed during ovalbumin aerosol exposure in OX40 knockout mice (Jember, et al. J.Exp.Med. 193, 387-392 (2001)). However, the kinetics of pathogen clearance with this fusion protein was not accelerated. In this model, therefore, OX40L:Ig treatment would be more beneficial than OX40:Ig.

Many studies have attempted to explain Th1 or Th2 development based on the level of co-stimulation received by the T cell including OX40 signalling (Lenschow, et al, Annu.Rev.Immunol. 14, 233-58, (1996); Fowell, et al. Immunity. 6, 559-569 (1997); Gause, et al, J.Immunol. 158,4082-4087 (1997)).. We now report that inhibition of OX40 co-stimulation interrupts both Th1 and Th2 responses during infection induced inflammatory disease. Treatment with OX40:Ig reduced IFNγ and TNF during lung viral infection and also Th2 dependent eosinophilic lung disease in mice infected with *C. neoformans.* In addition OX40L:Ig administration enhanced IFNγ and IL-4 during RSV infection. This supports previous studies showing that T cell differentiation is primarily dependent on the dose and affinity of peptide antigens rather than intrinsically reliant on co-stimulatory signals (Rogers & Croft. J.Immunol. 164, 2955-2963 (2000); Tanaka, et al. J Exp.Med. 192, 405-412 (2000)). Indeed OX40 ligand knockout mice show reduced Th1 and Th2 responses to protein and alloantigens (Murata, et al. J.Exp.Med. 191, 365-374 (2000)).

Our studies clearly show that harmful T cell immunity during lung influenza and RSV infection can be prevented by inhibiting OX40 co-stimulation (using OX40:Ig fusion proteins); an effect that does not prevent viral clearance or recall responses and is also beneficial after the onset of clinical symptoms. More importantly we show for the first time that infection induced lung eosinophilia can be prevented by 1) promoting Th1 development (using OX40L:Ig fusion protein) and 2) by reducing CD4⁺ T cells (using OX40:Ig fusion protein). In the case of OX40L:Ig administration this resulted in a reduced fungal burden. Manipulation of OX40 co-stimulation may therefore be beneficial for asthmatic and allergic conditions. Intervention of single co-stimulatory pathways may therefore be a realistic therapeutic strategy in the absence of suitable vaccines.

### Example 3

### Methods

### Mice and pathogens

8-12 week old female C57BL/6 mice (Harlan Olac Ltd, Bicester, UK) were kept in pathogen-free conditions according to Home Office guidelines. IFN-γ^{-/-} and IL-12^{-/-} p40 mice (back-crossed to C57BL/6 background at least 10 times) were purchased from The Jackson Laboratory (Bar Harbor, ME) and were maintained by homozygous matings under contract at Bantin & Kingman Universal. All mice came from specific pathogen-free colonies. *C. neoformans* strain 52, was obtained from the American Type Culture Collection (Rockville, USA) and for infection grown to stationary phase (48-72 hours) at room temperature on a shaker in Sabouraud dextrose broth (1% neopeptone and 2% dextrose; Difco, Detroit, MI). The cultures were washed in saline, counted on a haemocytometer and diluted in sterile nonpyrogenic saline to the required infective dose.

### Preparation of OX40 ligand fusion proteins

Murine OX40L:murine IgG1 fusion protein (OX40L:Ig) was constructed as described in Example 2 using a chimeric cDNA containing the C-terminal region of OX40L fused to the constant region of murine IgG1. LPS contamination of fusion protein was analysed by gas chromotography-mass spectrometry (GC-MS) of fatty acid methyl esters. Briefly, samples were derivatised using methanolic HCl and resulting fatty acid methyl esters were dissolved in hexanes prior to on column injection on a Stabilwax (30m x 0.25mm internal diameter, Restek Corp., PA) column. Samples were analysed using a temperature gradient of 150°C to 250°C at a rate of 3°C/min. Characteristic retention times and spectra of known standards were used to identify any fatty acid methyl esters present in the samples. All fusion protein used contained no LPS.

### Mouse infections and treatment

On day 0, mice were anaesthetised with halothane and intranasally infected with 2 x 10⁴ cfu *C.neoformans* in 50µl sterile PBS. Some groups of mice were injected intraperitoneally (i.p.) with 100 µg OX40L:Ig fusion proteins (Xenova pharmaceuticals, Cambridge, UK) or mouse IgG (Sigma-Aldric, Poole, Dorset) on various days after infection as indicated in the text. Mice were then sacrificed at various time points after C. neoformans infection by injection of 3 mg pentobarbitone and exsanguinated via the femoral vessels. Bronchoalveolar lavage fluid (BAL), lung tissue and sera were recovered using methods described previously.

### Cell recovery

Briefly, the lungs of each mouse were inflated 6 times with 1ml 1mM EDTA in EMEM and placed in sterile tubes on ice. 100 µl BAL fluid from each mouse was cytocentrifuged onto glass slides. The remainder was centrifuged and the supernatant removed and stored at -70°C in 200 µl aliquots for analysis of cytokines by ELISA. Cell viability was assessed using trypan blue exclusion and the pellet resuspended in RPMI containing 10 % FCS, 2 mM/ml L-glutamine, 50 U/ml penicillin and 50 µg/ml streptomycin (R10F) at a final concentration of 10⁶ cells/ml. Eosinophils were enumerated as granulocytes by flow cytometry, using forward and side scatter.

Identification was confirmed by counting eosinophils in H and E stained cytocentrifuge preparations.

### Flow cytometric analysis of intracellular and cell surface antigens

1 x 10⁶ BAL and lung derived cells were stained using APC-conjugated anti-CD4, anti-CD8-PerCP, anti-CD44-PE (all BD Pharmingen, Heidelberg, Germany) and anti-OX40-FITC (Serotec, Oxford, UK) for 30 min on ice. Cells were then fixed for 20 min at room temperature with 2 % formaldehyde. To detect intracellular cytokines, 10⁶ cells/ml were incubated with 50 ng/ml PMA (Sigma-Aldric, Poole, Dorset), 500 ng/ml ionomycin (Calbiochem, Nottingham, UK), and 10mg/ml brefeldin A (Sigma-Aldrich) for 4 hours at 37°C. Cells were then stained with anti-CD4-APC or anti-CD8-PerCP and fixed as described above. After permeabilization with PBS containing 1 % saponin/1 % BSA/0.05 % azide (saponin buffer) for 10 min FITC-conjugated anti-IFN-γ (Pharmingen) and PE-conjugated anti-IL5 (Pharmingen) diluted 1:50 in saponin buffer were added. 30 minutes later cells were washed once in saponin buffer and once in PBS/1 % BSA/0.1 % azide. All data was acquired on a FACSCalibur and analysed with CellQuest Pro software (BD Biosciences, Belgium). To set limits of background fluorescence appropriately labelled isotype matched control antibodies were used.

### Lung histology

In some studies, lungs were inflated and fixed with 10 % formalin in PBS, excised and embedded in paraffin wax by the histopathology department at the Hammersmith Hospital, UK. 4µm sections were stained with haematoxylin and eosin (H and E). The lungs from 4-5 mice per group were analysed.

### Enumeration of C. neoformans from lung homogenates

Lungs were homogenised by passage through 100 µm cell strainers (BD labware, New Jersey, USA). 100µl of cell suspension was diluted in PBS and incubated at room temperature for 48 hours on sabouraud dextrose agar plates (Sigma). The total colony forming units per lung was then determined (number of colonies x dilution factor x original cell suspension volume).

### Cryptococcus neoformans-specific antibody ELISA

2 x 10⁵ cfu/ml heat killed C.neoformans in PBS was used to coat 96 well microtiter plates overnight at room temperature on a shaker. After blocking with 3% BSA/PBS for 2 hours at 37°C, dilutions of sample sera were added for a further hour at room temperature. Bound antibody was detected using peroxidase-conjugated rabbit anti-mouse Ig and O-phenylene-diamine as a substrate. The reaction was stopped with 50 µl 2.5 M sulphuric acid. Optical densities were read at 490 nm and mean blank values (ODs from normal mouse serum) were subtracted from the optical density values of test samples.

### Immunohistochemical determination of cellular proliferation

*In vivo* incorporation of BrdU (Sigma) was determined to assess cellular proliferation in the lung. Mice were injected intraperitoneally with 75mg/kg BrdU 90 minutes before sacrifice. Lungs were inflated with 10% paraformaldehyde in PBS and embedded in paraffin. 4-µm sections were digested in 0.01% trypsin and then 1M hydrochloric acid. BrdU incorporation was detected with overnight incubation with a biotin labelled anti-BrdU monoclonal antibody (Caltag laboratories, South San Francisco, CA) followed by streptavidin peroxidase (Dako Corp., Carpinteria, CA) for 1 hour. Sections were then incubated with DAB substrate (Vector laboratories inc., Burlingame, CA) for 10 minutes. To compare proliferation between groups, sections were analysed microscopically, and the percentage of positively stained cells was counted in the perivascular regions in each sections. At least 400 cells were counted per section.

### Statistics

Statistical significance was evaluated using the student t test, 2 tailed assuming unequal variance within the Minitab software program.

### Results

### C. neoformans induces OX40 expressing T cells in the lung and airway.

C57BL/6 mice infected with C. neoformans develop extensive pulmonary eosinophilia that peaks around 11-14 days after infection. The kinetics of eosinophilia parallels that of C. neoformans cfu in the lung (Figure 15A). Peak recruitment of CD4⁺ T cells and to a lesser extent CD8⁺ T cells also occurs at times of maximal eosinophilia and pathogen burden (Figure 15B). As reported previously C57BL/6 mice do not completely clear the infection and low levels remained in the lung indefinitely.

Surface expression of OX40 is up-regulated by T cells 1-2 days after antigen stimulation. The uninfected mouse lung and airway did not contain any CD4+ T cells expressing OX40. Of the CD4+ T cells recruited to the lung during infection, however, up to 15% express OX40 (Figure 15C). Peak OX40 expression occurs first in the lung and then in the airway, which probably represents migration of T cells to the site of high pathogen burden. A representative dot plot of OX40 expression on CD4⁺ T cells is shown in figure 15D. This typical staining pattern shows that OX40 is expressed at various intensities on CD4⁺ T cells. Cells negative for CD4 but OX40+ are all CD8+ (data not shown).

### OX40L:Ig fusion protein enhances CD4+ T cell responses during C. neoformans infection.

After determining that OX40 was expressed by infiltrating T cells we then determined whether T cell responses could be influenced by OX40L:Ig fusion protein administration. In uninfected mice this fusion protein has no effect. Whilst not wishing to be bound by theory, we believe this is due to i) the low number of T cells in naive mice but more importantly ii) that those T cells present do not express OX40 until activated by antigen. In addition, administration of OX40L:Ig alone did not induce any noticeable change in the cellular composition in the BAL or lung (data not shown). Administration of OX40L:Ig to C. neoformans infected mice, however, increased the kinetics of accumulation of T cells in the lung (Figure 16a), which may represent increased survival and/or proliferation. Enhanced inflammatory infiltration can clearly be observed in H and E stained sections of lung. In untreated mice a minimal infiltrate is observed around the airways and blood vessels 11 days post-infection (Figure 16b). OX40L:Ig treatment significantly increases this inflammatory infiltration (Figure 16c).

To determine the effect on cell proliferation BrdU was administrated 90 minutes before termination of OX40L:Ig and control treated *C. neoformans* infected mice. Lungs were then inflation fixed, removed, embedded in paraffin and 4 µm sections stained with an anti-BrdU antibody. For 10 days after *C. neoformans* infection there was no discernible difference in the number of lung cells expressing BrdU. By day 11 however there were significantly more proliferating cells in the lungs of OX40L:Ig (Figure 17b) compared to control (Figure 17a) treated mice (Figure 17c). This difference persisted throughout the remainder of the time course.

### OX40L:Ig controls pathogen burden and reduces associated eosinophilia.

Since T cell numbers were increased we next determined the effect of OX40L:Ig fusion protein treatment on pathogen burden and associated eosinophilia. Similar colony forming units were recovered from infected mice during initial time points regardless of the treatment. From day 12 onwards, a significantly reduced pathogen burden was observed in OX40L:Ig treated mice compared to controls in three independent experiments (Figure 18a). The recruitment of eosinophils to the lung follows similar kinetics to that of the pathogen (i.e. both are minimal at early stages after infection and peak around days 12-15). OX40L:Ig treatment reduced the percent eosinophilia in the lung (Figure 18b).

### OX40L:Ig treatment increases IFN-γin CD4⁺ T cells.

Since eosinophilia in this model requires CD4⁺ T cells secreting type 2 cytokines, we next investigated the cytokine profile in treated mice by intracellular cytokine staining. Only a few lung CD4⁺ T cells expressed intracellular IFN-γ in control treated mice (Figure 19a). OX40L:Ig however increased this relative production 2-3 fold (Figure 19b) and total numbers were increased from 1.61 ± 1.34 x10⁴ cells in control mice to 11.67 ± 3.4 x 10⁴ cells in OX40L:Ig treated mice (p <0.05). By calculating the ratio of IFN-γ:IL-5 expressing cells we can see that OX40L:Ig treatment significantly shifts the CD4⁺ T cell cytokines in the lung to a Th1 phenotype (Figure 19c). Consistent with this we observed a reduction in IgE 7 days post-infection in nasal wash following OX40L:Ig treatment compared with control treated mice (4/5 mice, p<0.05).

### IFN-γis required for the reduction in pathogen burden and eosinophilia by OK40L:Ig

The mechanism by which OX40L:Ig mediates its effect was then investigated in IFN-γ and IL-12 knockout mice. Wild type immuno-competent mice produced similar results to previous experiments by showing increased CD4⁺ T cells (Figure 20a) but reduced pathogen burden (figure 20b) and eosinophils (figure 20c) after OX40L:Ig treatment. Experiments in the knockout mice produced surprising results. The absence of IFN-γ or IL-12 did not affect the OX40L:Ig induced increment in CD4+ T cell numbers (Figure 20a) or their activation (data not shown). The ability of OX40L:Ig to reduce pathogen burden (Figure 20b) and eosinophilia (Figure 20c) however was impaired and indistinguishable from control treated wild type groups. We originally thought that eosinophils might actually increase in both knockout mouse strains due to impaired IFN-γ production. This was not the case despite the low levels of IFN-γ and the small but significant increase in intracellular IL-5 production by CD4⁺ T cells in IL-12 knockout mice (Table 1).

| **Strain** | **Treatment** | **%CD4/ IFN-**γ | **Total CD4/ IFN-**γ **(x10⁻⁴)** | **%CD4/ IL-5** | **Total CD4/ IL-5 (x10⁻⁴)** |
|---|---|---|---|---|---|
| IFN-γ⁻⁴ | Control Ig | 0.105 ± 0.137 | 0.03 ± 0.03 | 3.1 ± 0.29 | 1.3 ± .055 |
| | OX40L:Ig | 0.15 ±0.3 | 0.008 ± 0.015 | 9.48 ± 8.22 | 2.35 ± 2.96 |
| IL.12^{-/-} | Control Ig | 1.36 ± 0.74 | 1.09 ± 0.34 | 5.9 ± 2.65 | 5.33 ± 2.57 |
| | OX40L:Ig | 3.6 ± 1.26* | 3.32 ± 1.62* | 12.9 ± 3.67* | 14.73 ± 10.74 |

### Discussion

The OX40:OX40 ligand axis presents a novel method of targeting activated T cells since OX40 is only induced after T cells receive two initial signals: TCR interaction with MHC class I or II bound peptide and the CD28:B7 interaction. There are a number of infectious diseases where protection is poor due to insufficient or transient immunity. The specific enhancement of responding T cells at the time of infection or vaccination in situ may therefore afford better protective immunity without disturbing the rest of the T cell repertoire. Current immune adjuvants enhance immune responses by increasing antigen uptake or the kinetics of inflammatory reactions. This can have unwanted side effects including immune recognition of the adjuvant and toxicity. Similarly immune reactions could be promoted using B7 fusion proteins, but this would affect naive as well as antigen activated T cells. We now report for the first time the beneficial effect of promoting T cells via OX40 engagement during an infectious disease using OX40 ligand fusion protein (OX40L:Ig). During C. neoformans infection OX40L:Ig administration promotes cell mediated immunity and IFN-γ production by CD4⁺ T cells, while reducing the pathogen burden in the lung and the associated eosinophilia. Such fusion proteins also have a longer half life (approximately 4 days) compared to antibody (17 hours) and can be constructed entirely of human proteins without the necessity for "humanisation" or more complex CDR3 grafting.

OX40L:Ig administration alone does not produce any visible effect in naive mice and yet has a profound influence when present during inflammation caused by *C. neoformans.* The critical trigger appears to be the level of OX40 expressed by the T cell, which is virtually absent in uninfected mice but increases on CD4⁺ T cells during infection. The next reasonable question to pose during *C. neoformans* infection would therefore be why doesn't the endogenous natural interaction between OX40 and OX40 ligand cause the same effect in the absence of the fusion protein. OX40 ligand expression, though constitutive on cardiac myocytes and a subset of DCs in SCID mice is limiting in the absence of a "danger signal". This signal can be provided by a strong adjuvant, such as complete Freunds adjuvant or LPS. Studies detailing the ability of infections to provide a sufficient OX40 ligand inducing signal to the APC are limiting. We show however that *C. neoformans* is equivalent to LPS in its ability to induce OX40 ligand. The beneficial effect of fusion protein administration is therefore likely to be the provision of increased engagement over and above that naturally provided *in vivo.* This hypothesis is supported by studies showing a three-fold increase in germinal centre T cells in transgenic mice over-expressing OX40 ligand in dendritic cells. Similarly, stimulation of OX40 using anti-OX40 antibody increases T cells specific for soluble, or super, antigens. Combined with LPS and a 60 fold increase is observed. An alternative explanation is that enhanced signals via OX40 using OX40L:Ig overrides the negative signals provided by CTLA-4. This hypothesis is important since *C. neoformans* induces T cell expression of CTLA-4 and anti-CTLA-4 treatment has been shown to enhance pathogen clearance.

The increase of CD4⁺ T cells by OX40L:Ig in our study is likely to reflect enhanced proliferation and/or survival due to the reported up-regulation of survival factors Bcl-2 and Bcl-x_{L}. Enhanced BrdU uptake by infiltrating cells was clearly observed in lung sections. It is interesting to note that this effect was only observed 12 days after *C. neoformans* infection. This time point is consistently associated with maximal pathogen burden and eosinophilia. There is therefore a lag phase during which T cells are recruited and antigen-activated before OX40L:Ig can exert its effect. Enhanced T cell responses by OX40 engagement have also been reported in TCR transgenic mice where responsiveness to antigen remained 95 days after initial antigen exposure and *in vitro* using OX40L transfected fibroblasts.

One concern during our studies was that OX40L:Ig would prevent extravasation of T cells into the lung since the ligand is also expressed by endothelial cells and mediates adhesion of activated T cells. If T cells expressed OX40 prior to exiting the blood then OX40:Ig may bind and prevent interaction with the native ligand on endothelial cells. The increment in T cell numbers in the lung suggests that this did not occur. This is probably due to the lack of OX40 expression on T cells prior to antigen activation in the lung itself.

The eosinophilic response during *C. neoformans* infection is dependent on T cells secreting type II cytokines. The reduction of eosinophils by OX40L:Ig treatment may therefore be a consequence of one or more of the following: 1) enhanced direct apoptosis by IFN-γ, 2) reduced IL-5 or 3) decreased T cell stimulation due to the lower pathogen burden. Whilst not wishing to be bound by theory, we believe option one is more likely since IFN-γ was increased 3 fold, T cell numbers were actually increased and eosinophils were not reduced in IFN-γ or IL-12 knockout mice infected with *C. neoformans* and treated with OX40L:Ig. Though we did not observe a change in intracellular IL-5 expression in CD4+ T cells the cytokine balance is definitely in favour of a Th1 environment. The induction of IFN-γ by enhanced OX40 signalling has been described previously in re-call responses to antigens encountered during OX-40 engagement. CD8⁺ intraepithelial lymphocytes expressing OX40 and OX40L after CD3 stimulation also produce high levels of IFN-γ. The cytokine retort by T cells after engagement of OX40 appears to alter depending on the cell population and the antigen being studied since other data shows effects on Th1 and/or Th2 cytokines.

The reduction of *C. neoformans* by OX40L:Ig is also likely to be dependent on enhanced IFN-γ, which activates macrophages and increases their fungicidal activity. CD4⁺ T cells play an important role in recruiting macrophages during virulent Cryptococcal infection. In our study we observed increased CD4⁺ T cells secreting IFN-γ and an increase in the number of macrophages (data not shown). This therefore provides an environment more equipped to manage fungal clearance. This idea is supported by the inability of OX40L:Ig to decrease cfus in IL-12 and IFN-γ knockout mice. CD4⁺ T cells expressing OX40 are also present in the lung earlier during OX40L:Ig treatment and may therefore assist macrophages in the early control of the pathogen. Previous studies have detailed the importance of IL-12 in protection from *C. neoformans.* Optimal production of IL-12 requires CD40 on T cells interacting with CD40L on APCs. Blockade of this interaction decreases IFN-γ and fungicidal activity. The presence of more T cells in OX40L:Ig treated mice may therefore enhance CD40:CD40L interactions.

Since we have demonstrated an alteration in the cytokine environment the next question was what has happened to antibody production? Since OX40:OX40L interactions are thought to play a role in antibody production, we initially thought that the OX40L:Ig treatment may block the signal to OX40L expressing B cells. Total B cell numbers and IgE production in the BAL were not affected (data not shown) in OX40L:Ig treated mice, presumably due to the presence of other B cell stimulatory signals such as CD40 and CD70. This is similar to the absence of any affect on B cell immunity reported in OX40 knockout mice.

It is interesting to note that OX40L:Ig still increased T cell numbers in both IL-12 and IFN-γ knockout mice. Such cytokine defects do not appear to prevent antigen presentation and T cell clonal expansion. IL-12 in addition to increasing IFN-γ is also reported to induce TNF-α, GM-CSF, IL-8 and even IL-10. These do not however appear to compensate for a lack of IFIV-γ in knockout mice.

In summary, this example shows for the first time that OX40 ligation can reduce the symptoms of a lung infection. This effect was dependent on IFN-γ and IL-12. Since OX40 is expressed on antigen activated T cells this represents a novel strategy to enhance immunity to infectious diseases. Specific enhancement in the number and function of T cells actually participating in an immune response has not previously been tested in an infectious disease. OX40L:Ig fusion proteins have been shown to enhance tumour immunity, This strategy therefore holds significant promise for the plethora of infections that induce insufficient immunity or latency.

The invention is susceptible of modifications and variations as will be apparent to those skilled in the art, and the present disclosure extends to combinations and subcombinations of the features mentioned or described herein and in the published documents referred to.

## Claims

1. A composition comprising one or more OX40 polypeptides or biologically active fragments thereof, or antibodies which bind to OX40, that can inhibit immune system costimulation by OX40, for use in treating, ameliorating or preventing virus induced immunopathology.

2. The composition as claimed in claim 1, wherein the virus induced immunopathology occurs in the lung.

3. The composition as claimed in claim 1 or claim 2, wherein the virus induced immunopathology is caused by influenza virus.

4. The composition as claimed in claim 1 or claim 2, wherein the virus induced immunopathology is caused by respiratory syncytial virus (RSV).

5. The composition as claimed in any preceding claim, wherein the OX40 polypeptide is a fusion protein based on OX40 and an immunoglobulin, preferably IgG.

6. The composition as claimed in claim 5, wherein the fusion protein comprises the extracellular domain of OX40 linked via its C-terminal to the N-terminal of the constant domain of an immunoglobulin.

7. The composition as claimed in claim 5 or claim 6, wherein the OX40 and immunoglobulin are from the same (human or non-human animal) species as the species to be treated,

8. A product containing the composition of any one of claims 1-7 and an anti-inflammatory drug as a combined preparation for simultaneous, sequential or separate use in treating, ameliorating or preventing virus induced immunopathology.

## Patentansprüche

1. Zusammensetzung, umfassend ein oder mehrere OX40-Polypeptide oder biologisch aktive Fragmente davon, oder Antikörper, welche an OX40 binden, die Immunsystem-Kostimulation durch OX40 hemmen kann, zur Verwendung beim Behandeln, Bessern oder Verhindern von Virus-induzierter Immunpathologie.

2. Zusammensetzung wie in Anspruch 1 beansprucht, wobei sich die Virus-induzierte Immunpathologie in der Lunge ereignet.

3. Zusammensetzung wie in Anspruch 1 oder Anspruch 2 beansprucht, wobei die Virus-induzierte Immunpathologie durch Influenzavirus verursacht wird.

4. Zusammensetzung wie in Anspruch 1 oder Anspruch 2 beansprucht, wobei die Virus-induzierte Immunpathologie durch respiratorisches Synzytial-virus (RSV) verursacht wird.

5. Zusammensetzung wie in einem vorhergehenden Anspruch beansprucht, worin das OX40-Polypeptid ein Fusionsprotein ist, basierend auf OX40 und einem Immunglobulin, vorzugsweise IgG.

6. Zusammensetzung wie in Anspruch 5 beansprucht, worin das Fusionsprotein die extrazelluläre Domäne von OX40, gebunden über sein C-terminales Ende an das N-terminale Ende der konstanten Domäne eines Immunglobulins, umfasst.

7. Zusammensetzung wie in Anspruch 5 oder Anspruch 6 beansprucht, worin das OX40 und Immunglobulin von der gleichen (Mensch oder nicht-menschliches Tier) Art sind, wie die zu behandelnde Art.

8. Produkt, welches die Zusammensetzung nach einem der Ansprüche 1-7 und eine entzündungshemmende Arznei als ein kombiniertes Präparat für gleichzeitige, sequentielle oder getrennte Verwendung beim Behandeln, Bessern oder Verhindern von Virus-induzierter Immunpathologie enthält.

## Revendications

1. Composition comprenant un ou plusieurs polypeptides OX40 ou ses/leurs fragments biologiquement actifs, ou anticorps qui se lient à OX40, qui peuvent inhiber une costimulation du système immunitaire par OX40, pour une utilisation dans le traitement, le soulagement ou la prévention d'une immunopathologie induite par virus.

2. Composition selon la revendication 1, dans laquelle l'immunopathologie induite par virus survient dans les poumons.

3. Composition selon la revendication 1 ou la revendication 2, dans laquelle l'immunopathologie induite par virus est provoquée par un virus grippal.

4. Composition selon la revendication 1 ou la revendication 2, dans laquelle l'immunopathologie induite par virus est provoquée par un virus respiratoire syncytial (RSV).

5. Composition selon une quelconque revendication précédente, dans laquelle le polypeptide OX40 est une protéine hybride basée sur OX40 et une immunoglobuline, de préférence IgG.

6. Composition de la revendication 5, dans laquelle la protéine hybride comprend un domaine extracellulaire de OX40 lié par son C-terminal au N-terminal du domaine constant d'une immunoglobuline.

7. Composition selon la revendication 5 ou la revendication 6, dans laquelle le OX40 et l'immunoglobuline proviennent de la même espèce (animale humaine ou non-humaine) que l'espèce à traiter.

8. Produit contenant la composition selon l'une quelconque des revendications 1 à 7 et un médicament anti-inflammatoire en tant que préparation combinée pour une utilisation simultanée, séquentielle ou séparée dans le traitement, le soulagement ou la prévention d'une immunopathologie induite par virus.
